# EUROPEAN PATENT APPLICATION

(11) **EP 4 620 455 A1**
(43) Date of publication of application: **24.09.2025**
(21) Application number: 23891462.6
(22) Date of filing: 09.11.2023
(51) Int. Cl.: A61K 8/891, A61K 8/894, A61Q 1/04, A61Q 1/10, A61Q 1/12, A61Q 5/00, A61Q 5/06, A61Q 5/12, A61Q 17/04, A61Q 19/00

(54) **COSMETIC HAVING CROSSLINKED ORGANOSILOXANE POLYMER**

(30) Priority: 17.11.2022 JP 2022184419; 15.06.2023 JP 2023098638
(71) Applicant: SHIN-ETSU CHEMICAL CO., LTD., Tokyo 1000005 (JP)
(72) Inventor: AKABANE Emi, Annaka-shi, Gunma 379-0224 (JP); GOTO Tomoyuki, Annaka-shi, Gunma 379-0224 (JP)
(74) Representative: V.O.
(86) International application number: PCT/JP2023/040457
(87) International publication number: WO 2024/106312

(57) **Abstract**

[Problem]

The present invention has an object to improve the texture at the time of application by blending a crosslinked organosiloxane polymer that is non-fluid at 25 °C and has specific rheological characteristics into the cosmetic.

[Solution]

A cosmetic comprising a crosslinked organosiloxane polymer having a crosslinked structure including a carbon-silicon bond, which is non-fluid at 25 °C and has: a loss coefficient of 1.1 or less at a shear frequency of 1 Hz, a measurement temperature of 25 °C and a shear strain of 10 %; and a loss coefficient of 0.9 to 1.4 at a shear frequency of 10 Hz, a measurement temperature of 25 °C and a shear strain of 10 %.

## Description

### TECHNICAL FIELD

The present invention relates to cosmetics and personal care products using a crosslinked organosiloxane polymer that has specific rheological characteristics.

### BACKGROUND OF THE INVENTION

As a method to stably blend oil agents such as silicone oils, hydrocarbon oils, and ester oils into cosmetics and personal care compositions, organosiloxane polymers that have a crosslinked structure have been proposed. These are already known technologies, and various types of crosslinked organosiloxane polymers have been proposed. The crosslinked organosiloxane polymers are, for example, obtained by reacting a hydrogen siloxane with a diene compound or a diallyl compound. In this case, the crosslinked organosiloxane polymers can also be produced by reacting them in the oil agents or other solvents (Patent Literatures 1 to 7).

These crosslinked organosiloxane polymers are also known to provide high thixotropy and are also known to contribute to thickening effects and stability and provide a non-sticky feeling of coating thickness after application with regard to the cosmetic. However, their excessively high thixotropy causes a significant change in viscosity when applying force, leading to a sudden drop in viscosity at the time of application to the skin, in some cases causing users to feel poor lubricity at the beginning of application. These crosslinked organosiloxane polymers are resins or high-viscosity gums and are solids with high elasticity, in some cases causing users to feel difficulty in spreading on the skin or heaviness at the time of application. In addition, the crosslinked organosiloxane polymers are resins or high-viscosity gums and are solids with high elasticity, making it difficult to blend them homogeneously into the cosmetic in their original form and requiring them to be mixed with some of the oil agents to be blended into the cosmetic in advance to turn them into a liquid, paste, or gel, which is also a problem in terms of usability.

To solve the problems above, a liquid organopolysiloxane that has a micro-crosslinked structure has been proposed (Patent Literature 8). The liquid organopolysiloxane described in Patent Literature 8 can be easily blended and mixed homogeneously and stably with other liquid oils since it is a liquid, but there is still room for improvement in terms of texture. Patent Literature 9 proposes a method to improve the texture and uniformity by specifying the range of the loss coefficient tan(δ) of the liquid organopolysiloxane at a shear frequency of 10 Hz (Patent Literature 9). However, the method does not fully address the constantly changing texture at the time of the application of the cosmetic, and it cannot be said that the problems have been solved.

### PRIOR LITERATURES

### PATENT LITERATURES

Patent Literature 1 : Japanese Patent Application Laid-Open No. Hei 01-207354/1989
Patent Literature 2: Japanese Patent Application Laid-Open No. Hei 04-272932/1992
Patent Literature 3:WO2003/024413
Patent Literature 4:WO2004/024798
Patent Literature 5: Japanese Patent Application Laid-Open No. Hei 02-043263/1990
Patent Literature 6: Japanese Patent Application Laid-Open No. Hei 11-292972/1999
Patent Literature 7: Japanese Patent Application Laid-Open No. 2009-185296
Patent Literature 8: Japanese Patent Application Laid-Open No. 2012-246446
Patent Literature 9: Japanese Patent Application Laid-Open No. 2012-149052

### DISCLOSURE OF THE INVENTION

### PROBLEMS TO BE SOLVED BY THE INVENTION

The present invention was made in view of the problems above and has an object to improve the texture at the time of application by blending a crosslinked organosiloxane polymer that is non-fluid at 25 °C and has specific rheological characteristics into the cosmetic.

As a result of intensive studies to achieve the aforesaid object, the present inventors found that the object is achieved with an organosiloxane polymer that has specific rheological characteristics, leading to the completion of the present invention. That is, they found that the aforesaid object is achieved by blending, into a cosmetic, a crosslinked organosiloxane polymer having a crosslinked structure including a carbon-silicon bond, which is non-fluid at 25 °C and has a loss coefficient (Tδ) measured at different shear frequencies of 1.1 or less at a shear frequency of 1 Hz and of 0.9 to 1.4 at a shear frequency of 10 Hz, in which the loss coefficient is determined at a measurement temperature of 25 °C and a shear strain of 10 %.

The crosslinked organosiloxane polymer that has the rheological characteristics not only provides a non-sticky, moderate feeling of coating thickness after the application of the cosmetic but also offers a high degree of satisfaction with lubricity and good adhesion at the beginning of application and at the time of application.

The crosslinked organosiloxane polymer of the present invention is characterized by having the specific rheological characteristics. The types of functional groups and the molecular structure of the crosslinked organosiloxane polymer are not particularly limited as long as that the crosslinked organosiloxane polymer has a crosslinked structure including a carbon-silicon bond, is non-fluid at 25°C, and meet the rheological characteristics of the present invention.

The crosslinked organosiloxane polymer is preferably a product of a hydrosilylated reaction of one or more kinds of organohydrogen polysiloxanes with one or more kinds of alkenyl-group-containing organic compounds, wherein the organohydrogen polysiloxanes preferably have 2 to 10,000 silicon atoms and 1.01 to 10 hydroxy groups per molecule of the organohydrogen polysiloxane, and the alkenyl-group-containing compounds preferably have a weight average molecular weight of 50 to 9,000,000 and 1.01 to 10 alkenyl groups per molecule of the alkenyl-group-containing compound. The use of such organohydrogen polysiloxanes and alkenyl-group-containing organic compounds makes it easier to adjust the degree of crosslinking in the resulting crosslinked organosiloxane polymer and to obtain specific rheological characteristics.

The organohydrogen polysiloxane is preferably (A1) an organohydrogen polysiloxane represented by the following general formula (1).

Mₐ₁M'ₐ₂D_{b1}D'_{b2}T_{c1}T'_{c2}Q_{d} (1)

In formula (1),
M is a siloxane unit represented by R¹₃SiO_{1/2};
M' is a siloxane unit represented by R¹₂HSiO_{1/2};
D is a siloxane unit represented by R¹₂SiO_{2/2};
D' is a siloxane unit represented by R¹HSiO_{2/2};
T is a siloxane unit represented by R¹SiO_{3/2};
T' is a siloxane unit represented by HSiO_{3/2}; and
Q is a siloxane unit represented by SiO_{4/2}.

R¹ is, independently of each other, a group selected from an alkyl group having 1 to 30 carbon atoms, an aryl group having 6 to 30 carbon atoms, and an aralkyl group having 7 to 30 carbon atoms; and a1, a2, b1, b2, c1, c2, and d satisfy 0 ≤ a1 ≤ 100, 0 ≤ a2 ≤ 10, 0 ≤ b1 ≤ 10,000, 0 ≤ b2 ≤ 10, 0 ≤ c1 ≤ 100, 0 ≤ c2 ≤ 10, and 0 ≤ d ≤ 50, respectively, as well as 2 ≤ a1 + a2 + b1 + b2 + c1 + c2 + d ≤ 10,000 and 1.01 ≤ a2 + b2 + c2 ≤ 10.

Note that the organohydrogen polysiloxane may optionally include an organohydrogen polysiloxane represented by the general formula (1) of which a2 + b2 + c2 is 1, in addition to the organohydrogen polysiloxane represented by the general formula (1) as described above.

The alkenyl-group-containing organic compound is preferably one or more kinds of alkenyl-group-containing compounds selected from the following components (A2), (A3), (A4), and (A5).

(A2) Alkenyl-group-having organopolysiloxane represented by the following general formula (2):

Mₑ₁M"ₑ₂D_{f1}D"_{f2}T_{g1}T"_{g2}Qₕ (2)

In formula (2), M, D, T, and Q are the same as described above;
M" is a siloxane unit represented by R¹₂R²SiO_{1/2};
D" is a siloxane unit represented by R¹R²SiO_{2/2}; and
T" is a siloxane unit represented by R²SiO_{3/2};

R¹ is, independently of each other, a group selected from an alkyl group having 1 to 30 carbon atoms, an aryl group having 6 to 30 carbon atoms, and an aralkyl group having 7 to 30 carbon atoms; R² is an alkenyl group having 2 to 10 carbon atoms; and e1, e2, f1, f2, g1, g2, and h satisfy 0 ≤ e1 ≤ 100, 0 ≤ e2 ≤ 10, 0 ≤ f1 ≤ 100,000, 0 ≤ f2 ≤ 10, 0 ≤ g1 ≤ 100, 0 ≤ g2 ≤ 10, and 0 ≤ h ≤ 50, respectively, as well as 2 ≤ e1 + e2 + f1 + f2 + g1 + g2 + h ≤ 100,000 and 1.01 ≤ e2 + f2 + g2 ≤ 10.

Note that the alkenyl-group-having organopolysiloxane (A2) may optionally comprise an alkenyl-group-containing organopolysiloxane represented by the general formula (2) of which e2 + f2 + g2 is 1 in addition to the alkenyl-group-containing organopolysiloxane represented by the general formula (2) as described above.

(A3) Alkenyl-group-having oxyalkylene with alkenyl groups at both terminals represented by the following general formula (3):

CH₂=CH-(CH₂)ᵢ-(OC₂H₄)ⱼ-(OCH(CH₃)CH₂)ₖ-O-(CH₂)ᵢ-CH=CH₂ (3)

In the formula, i is, independently of each other, an integer of 1 or more, 1 ≤ j ≤ 100 and 0 ≤ k ≤ 100.

(A4) Alkene represented by the following general formula (4):

CH₂=CH-(CH₂)ᵣCH=CH₂ (4)

In the formula, 0 ≤ r ≤ 50.

(AS) Allyl-group-containing polyglycerin derivative represented by the following general formula (5):

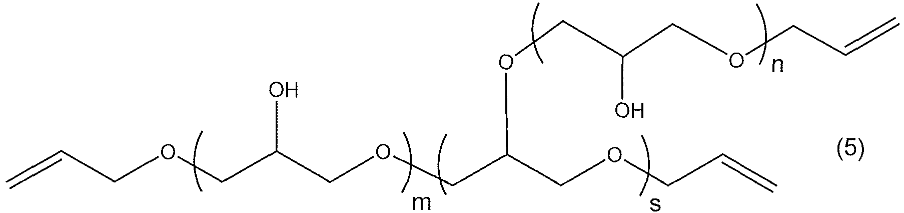

**In** the formula, 1 ≤ m ≤ 20, 1 ≤ n ≤ 20, and 0 ≤ s ≤ 10.

The preferable organohydrogen polysiloxanes and alkenyl-group-containing organic compounds described above are preferable as they smoothly promote polymerization and provide a crosslinked organosiloxane polymer having specific rheological characteristics of the present invention.

In addition, the crosslinked organosiloxane polymer of the present invention is more easily blended into a cosmetic by homogeneously mixing the crosslinked organosiloxane polymer with an oil agent (B) that is liquid at 25°C in advance.

### EFFECTS OF THE INVENTION

The cosmetic into which the crosslinked organosiloxane polymer of the present invention is blended is one whose texture obtained after application is improved. It is expected to provide a cosmetic that has a good texture, such as moderate lubricity and adhesion as well as a feeling of uniform coating thickness, from the beginning of application to after application. For example, it is suitably blended into skin care products, makeup products, UV-protection products, hair products, antiperspirant products, and deodorant products.

### DETAILED DESCRIPTION OF THE INVENTION

The present invention is described in more detail below.

### (A) Crosslinked organosiloxane polymer

The crosslinked organosiloxane polymer of the present invention is characterized by being non-fluid at 25°C. In the present invention, "non-fluid" means that the material is subjected to the solid-or-liquid determination test described below and determined to be a solid.

### [Solid-or-liquid determination]

Mark lines at 25 mm and 40 mm from the bottom of a 20 mL glass screw tube (LABORAN Screw Tube Bottle No. 5 with a body diameter of 27 mm, manufactured by AS ONE CORPORATION) and inject a sample up to the line marked at 25 mm. Next, seal the sample and leave it to stand at a sustained temperature in a thermostatic chamber at 25 °C for 24 hours. Then, tilt the screw tube horizontally. The sample is determined to be "a liquid" if the tip of the sample reaches the line marked at 40 mm within 96 hours or "a solid" if the tip of the sample does not reach the line.

The crosslinked organosiloxane polymer of the present invention is characterized by having a crosslinked structure including a carbon-silicon bond and a loss coefficient measured at different shear frequencies at a measurement temperature of 25°C and a shear strain of 10% of 1.1 or less at a shear frequency of 1 Hz and of 0.9 to 1.4 at a shear frequency of 10 Hz. The loss coefficient, also referred to as the loss tangent, is expressed as tan(δ). This value is calculated as the ratio of the elastic loss modulus (G") to the elastic storage modulus (G'), G"/G', and represents how much energy is absorbed or converted into heat when an object deforms. The loss coefficient may be measured with a dynamic viscoelasticity measuring device. Several types of dynamic viscoelasticity measuring devices are available to calculate each of the parameters by applying oscillatory strain such as tension, compression, bending, and shear to the object. The type of dynamic viscoelasticity measuring device is selected according to the shape and the purpose of the object, with the loss coefficient of the present invention being the value calculated using a rotational rheometer. In the measurement, a set strain value within the range of 0.01% to 10% does not significantly change the results, but 1% to 10% is preferable, and, in this case, a strain value of 10% was set as a measurement condition.

In the measurement, a set gap within the range of 0.2 mm to 1.5 mm does not significantly change the results, but 0.5 mm to 1.0 mm is preferable, and, in this case, a gap of 0.5 mm was set as a measurement condition.

In general, for viscoelastic materials, the higher the elasticity, the lower the loss coefficient. Such materials primarily have a three-dimensional crosslinked structure. On the other hand, high-viscosity materials, particularly those that are fluid, have a large loss coefficient, and for low-viscosity liquids that have a straight-chain structure, the loss coefficient cannot be measured because no torque can be applied. A loss coefficient value close to 1 means that the elasticity and viscosity are moderately balanced.

The organosiloxane polymer of the present invention preferably has a moderately low crosslink density or a crosslinked structure in part of its structure. The organosiloxane polymer with a preferably controlled crosslink density has a loss coefficient in the present ranges described above and strike a balance between its elasticity and viscosity.

Although the crosslinked organosiloxane polymer of the present invention has a loss coefficient of 1.1 or less at a shear frequency of 1 Hz, the loss coefficient tends to increase as the shear frequency increases, up to a certain shear frequency value. When comparing the loss coefficients at 1 Hz and 10 Hz, the value measured at 10 Hz tends to be slightly higher, which suggests that the repulsive force decreases as the shear frequency increases.

If the loss coefficient at a shear frequency of 1 Hz is higher than 1.1 and the loss coefficient at a shear frequency of 10 Hz is higher than 1.4, the polymer is fluid and, when blended into the cosmetic, has excessive lubricity, resulting in an unpleasant slimy texture or difficulty in feeling sufficient coating thickness. On the other hand, if the loss coefficient at a shear frequency of 1 Hz is 1.1 or less and the loss coefficient at a shear frequency of 10 Hz is less than 0.9, the elasticity increases, and, when blended into the cosmetic, it fails to obtain good lubricity and tends to cause a feeling of difficulty in application.

In the present invention, the structure of the crosslinked organosiloxane polymer is not particularly limited as long as the crosslinked organosiloxane polymer has a crosslinked structure including a carbon-silicon bond, as described above.

The crosslinked organosiloxane polymer is preferably a product of a hydrosilylated reaction with one or more kinds of organohydrogen polysiloxanes and one or more kinds of alkenyl-group-containing organic compounds, wherein the organohydrogen polysiloxanes preferably have 2 to 10,000 silicon atoms and 1.01 to 10 hydroxy groups per molecule of the organohydrogen polysiloxanes, and the alkenyl-group-containing compounds preferably have a weight average molecular weight of 50 to 9,000,000 and 1.01 to 10 alkenyl groups per molecule of the alkenyl-group-containing compounds. More preferably, the organohydrogen polysiloxanes have silicon atoms of 3 to 5,000 and hydroxy groups of 1.01 to 8, further preferably 1.01 to 5 per molecule of the organohydrogen polysiloxanes. The alkenyl-group-containing compounds more preferably have a weight average molecular weight of 50 to 900,000 and alkenyl groups of 1.01 to 8, further preferably 1.01 to 5 per molecule of the alkenyl-group-containing compounds.

Note that in the present invention, the weight average molecular weight of the organopolysiloxane is measured with gel permeation chromatography (GPC) analysis using polystyrene as the standard material, but if, for example, it may be calculated from its structure such as a pentadiene, octadiene, or decadiene corresponding to the alkene (A4), the molecular weight calculated from the structure shall be used instead of the weight average molecular weight.

The organohydrogen polysiloxanes may optionally comprise an organohydrogen polysiloxane that has two or more hydroxy groups per molecule as well as an organohydrogen polysiloxane that has only one hydroxy group per molecule as long as the number of hydroxyl groups per molecule of the organohydrogen polysiloxanes is within the range of 1.01 to 10. The alkenyl-group-having organopolysiloxanes may also optionally comprise an alkenyl-group-containing organopolysiloxane that has two or more alkenyl groups per molecule as well as an alkenyl-group-containing organopolysiloxane that has only one alkenyl group per molecule as long as the number of alkenyl groups per molecule of the alkenyl-group-containing compounds is within the range of 1.01 to 10. That is, when the total number of hydroxy groups in the organohydrogen polysiloxanes is less than two on average per molecule of the organohydrogen polysiloxanes, a crosslinkable organohydrogen polysiloxane and a non-crosslinkable organohydrogen polysiloxane are mixed in combination. Similarly, when the number of alkenyl groups in the alkenyl-group-containing organic compounds is less than two on average per molecule of the alkenyl-group-containing compounds, a crosslinkable alkenyl-group-containing organic compound and a non-crosslinkable alkenyl-group-containing compound are mixed in combination.

The specific rheological characteristics of the polymer of the present invention may be obtained with the crosslinked polymer described above. The degree of crosslinking is required to be appropriately adjusted depending on the types of the organohydrogen polysiloxanes and the alkenyl-group-containing organic compounds. A plurality of kinds of organohydrogen polysiloxanes and a plurality of kinds of alkenyl-group-containing compounds may also be combined as needed. The results of the studies conducted by the present inventors suggest that the fewer the number of crosslinking points resulting from the hydrosilylation reaction, the more preferable. Thus, if the total number of hydroxy groups involved in the formation of the crosslinked structure is more than 10 per mole of the organohydrogen polysiloxanes, the resulting polymer may be hard and be a powder without any aggregation. If the total number of hydroxy groups is less than 1.01 per mole of the organohydrogen polysiloxanes, it may have almost no crosslinked structure and be a liquid with low viscosity and a loss coefficient that is high or unmeasurable. If the total number of alkenyl groups is 10 or more on average per mole of the alkenyl-group-containing compounds, the polymer may similarly be hard, and if it is less than 1.01 on average per mole, the polymer may be more likely to be a liquid. Note that when two or more organohydrogen polysiloxanes are used in combination, the number per mole is calculated using the method described below.

For example, if 1 mole of an organohydrogen polysiloxane having two hydroxy groups in its structure and 0.5 moles of an organohydrogen polysiloxane having five hydroxy groups in its structure are used in combination, the number of hydroxy groups per mole of the hydrogen polysiloxanes is calculated to be 3.0. Similarly, the same method is used for the alkenyl-group-containing organic compounds.

The structure of the crosslinked organosiloxane polymer is not particularly limited and is only required to be a crosslinked structure including a carbon-silicon bond. For example, it is preferably a reaction product of one or more of the organohydrogen siloxane (A1) selected from the compounds shown below and one or more alkenyl-group-containing organic compounds selected from the following components (A2), (A3), (A4) and (AS) having an alkenyl group.

(A1) is an organohydrogen polysiloxane having a hydrogen atom bonded to a silicon atom represented by the following general formula (1).

Mₐ₁M'ₐ₂D_{b1}D'_{b2}T_{c1}T'_{c2}Q_{d} (1)

In formula (1),
M is a siloxane unit represented by R¹₃SiO_{1/2};
M' is a siloxane unit represented by R¹₂HSiO_{1/2};
D is a siloxane unit represented by R¹₂SiO_{2/2};
D' is a siloxane unit represented by R¹HSiO_{2/2};
T is a siloxane unit represented by R¹SiO_{3/2};
T' is a siloxane unit represented by HSiO_{3/2}; and
Q is a siloxane unit represented by SiO_{4/2}.

In the above, R¹ is, independently of each other, a group selected from an alkyl group having 1 to 30 carbon atoms or preferably 1 to 16 carbon atoms, an aryl group having 6 to 30 carbon atoms or preferably 6 to 10 carbon atoms, and an aralkyl group having 7 to 30 carbon atoms or preferably 7 to 10 carbon atoms; and a1, a2, b1, b2, c1, c2, and d satisfy 0 ≤ a1 ≤ 100, 0 ≤ a2 ≤ 10, 0 ≤ b1 ≤ 10,000, 0 ≤ b2 ≤ 10, 0 ≤ c1 ≤ 100, 0 ≤ c2 ≤ 10, and 0 ≤ d ≤ 50, respectively, as well as 2 ≤ a1 + a2 + b1 + b2 + c1 + c2 + d ≤ 10,000 and 1.01 ≤ a2 + b2 + c2 ≤ 10.

Note that the organohydrogen polysiloxane may optionally comprise an organohydrogen polysiloxane represented by the general formula (1) of which a2 + b2 + c2 is 1 in addition to the organohydrogen polysiloxane represented by the general formula (1) as described above.

Examples of the alkyl groups represented by R¹ include a methyl group, an ethyl group, a propyl group, an isopropyl group, a butyl group, an isobutyl group, a tert-butyl group, a pentyl group, a neopentyl group, a hexyl group, a heptyl group, an octyl group, a nonyl group, a decyl group, and a dodecyl group. It is preferably a methyl group, an ethyl group, a propyl group, an isopropyl group, a butyl group, a hexyl group, or an octyl group, and a methyl group is particularly preferable. Examples of the aryl groups include a phenyl group, a tolyl group, a xylyl group, and a naphthyl group, and a phenyl group is particularly preferable among these groups. Examples of the aralkyl groups include a benzyl group, a phenylethyl group, a phenylpropyl group, and a methylbenzyl group, and a benzyl group or a phenylpropyl group is preferable.

a1, a2, b1, b2, c1, c2, and d are as described above: a1 is an integer of 0 to 100, preferably an integer of 0 to 50, or more preferably 0 or an integer of 1 to 20; a2 is an integer of 0 to 10 or preferably an integer of 0 to 8; b1 is an integer of 0 to 10,000, preferably an integer of 0 to 1,000, more preferably an integer of 1 to 500, even more preferably an integer of 2 to 200, or still more preferably an integer of 3 to 100; b2 is an integer of 0 to 10, or preferably 0 or an integer of 1 to 8; c1 is an integer of 0 to 100, preferably an integer of 0 to 50, or more preferably 0 or an integer of 1 to 30; c2 is an integer of 0 to 10 or preferably an integer of 0 to 8; and d is an integer of 0 to 50 or preferably an integer of 0 to 20.

For the organohydrogen polysiloxane represented by the formula (1), a2 + b2 + c2 is 1.01 or more and 10 or less, preferably 1.01 or more and 8 or less, or more preferably 1.02 or more and 5 or less. a1 + a2 + b1 + b2 + c1 + c2 + d is 2 or more and 10,000 or less, preferably 3 or more and 5,000 or less, or more preferably 4 or more and 1,000 or less.

The organohydrogen polysiloxane of the formula (1) may be used alone or in combination with a plurality of types. The organohydrogen polysiloxane wherein a2 + b2 + c2 is 1 can also be used in combination.

The following general formula (2) is an alkenyl-group-having organopolysiloxane.

Mₑ₁M"ₑ₂D_{f1}D"_{f2}T_{g1}T"_{g2}Qₕ (2)

In formula (2), M, D, T, and Q are the same as described above;
M" is R¹₂R²SiO_{1/2};
D" is a siloxane unit represented by R¹R²SiO_{2/2}; and
T" is a siloxane unit represented by R²SiO_{3/2}.
R¹ is, independently of each other, a group selected from an alkyl group having 1 to 30 carbon atoms, an aryl group having 6 to 30 carbon atoms, and an aralkyl group having 7 to 30 carbon atoms, and the examples given for the formula (1) are applied for R¹.
R² is an alkenyl group having 2 to 10 carbon atoms or preferably 2 to 6 carbon atoms; and e1, e2, f1, f2, g1, g2, and h satisfy 0 ≤ e1 ≤ 100, 0 ≤ e2 ≤ 10, 0 ≤ f1 ≤ 100,000, 0 ≤ f2 ≤ 10, 0 ≤ g1 ≤ 100, 0 ≤ g2 ≤ 10, and 0 ≤ h ≤ 50, respectively, as well as 2 ≤ e1 + e2 + f1 + f2 + g1 + g2 + h ≤ 100,000 and 1.01 ≤ e2 + f2 + g2 ≤ 10.

Note that the alkenyl-group-having organopolysiloxane (A2) may optionally comprise an alkenyl-group-containing organopolysiloxane represented by the general formula (2) of which e2 + f2 + g2 is 1 in addition to the alkenyl-group-having organopolysiloxane represented by the general formula (2) as described above.

In the general formula (2), the definition for R¹ in the formula (1) applies to R¹. R² is an alkenyl group having 2 to 10 carbon atoms. Examples of the alkenyl group include a vinyl group, an allyl group, a propenyl group, a butenyl group, a pentenyl group, and a hexenyl group. A vinyl group is preferable because of the ease of hydrosilylation reactions with hydrogen atoms bonded to silicon and its stability.

In the general formula (2), e1 is an integer of 0 to 100, preferably an integer of 0 to 50, or more preferably 0 or an integer of 1 to 30; e2 is an integer of 0 to 10, or preferably 0 or an integer of 1 to 8; f1 is an integer of 0 to 1,000, preferably an integer of 1 to 500, more preferably an integer of 2 to 200, or even more preferably an integer of 3 to 100; f2 is an integer of 0 to 10, or preferably 0 or an integer of 1 to 8; g1 is an integer of 0 to 100, preferably an integer of 0 to 50, or more preferably 0 or an integer of 1 to 30; g2 is an integer of 0 to 10 or preferably an integer of 0 to 8; and h is an integer of 0 to 50 or preferably an integer of 0 to 20.

e2 + f2 + g2 in the general formula (2) is 1.01 or more and 10 or less, preferably 1.01 or more and 8 or less, or more preferably 1.02 or more and 5 or less. e1 + e2 + f1 + f2 + g1 + g2 + h is 2 or more and 100,000 or less, preferably 2 or more and 10,000 or less, or more preferably 2 or more and 1,000 or less.

The organopolysiloxane (A2) may be used alone or in combination with a plurality of types. The organopolysiloxane wherein e2 + f2 + g2 is 1 may also be used in combination. Relative to the entire alkenyl-group-containing organopolysiloxane, Component (A2) of the present invention has a weight average molecular weight of 50 to 9,000,000, preferably a weight average molecular weight of 50 to 900,000, and has 1.01 to 10, preferably 1.01 to 8, or more preferably 1.01 to 5 alkenyl groups per mole of the alkenyl-group-containing organopolysiloxane. The blending ratio of two or more alkenyl-group-containing organopolysiloxanes is appropriately adjusted to be within the range.

The methods for producing the organohydrogen polysiloxane (A1) and the alkenyl-group-containing organopolysiloxane (A2) are not particularly limited. In general, they are produced by an equilibration reaction with an acid catalyst or an alkali catalyst using siloxanes consisting mainly of cyclic and/or acyclic siloxane oligomers obtained by hydrolyzing dichlorodimethylsilane or further cracking the hydrolysate in the presence of an alkali catalyst such as potassium hydroxide as ingredients. The resulting polysiloxanes may also be subjected to treatment such as vacuum stripping.

(A3) is a polyoxyalkylene that has alkenyl groups at both terminals and represented by the following general formula (3) .

CH₂=CH-(CH₂)ᵢ-(OC₂H₄)ⱼ-(OCH(CH₃)CH₂)ₖ-O-(CH₂)ᵢ-CH=CH₂ (3)

In the formula, i is, independently of each other, an integer of 1 or more, 1 ≤ j ≤ 100 and 0 ≤ k ≤ 100.

In the general formula (3), i may be the same or different and is an integer of 1 or more, preferably 1 to 10, or more preferably 1 to 5; j is 1 or more and 100 or less, preferably 2 to 100, more preferably 3 to 80, or even more preferably 4 to 30; and k is 0 or more, preferably 0 to 100, or more preferably 0 to 80.

The method for producing the polyoxyalkylene (A3) is already known and is not particularly limited. For example, it may be produced by ring-opening alkylene oxide in the presence of an alkali catalyst, addition polymerizing the resultant to obtain a polymer, and then reacting an alkenyl-group-containing halide at a terminal of the polymer. The polyoxyalkylene (A3) may be used alone or in combination with a plurality of kinds.

(A4) is an alkene represented by the following general formula (4).

CH₂=CH-(CH₂)ᵣ-CH=CH₂ (4)

In the general formula (4), r is 0 or more to 50 or less, preferably 1 to 20, more preferably 1 to 15.

The alkene (A4) may be used alone or as a mixture of a plurality of kinds.

(A5) is an allyl-group-containing polyglycerin derivative represented by the following general formula (5).

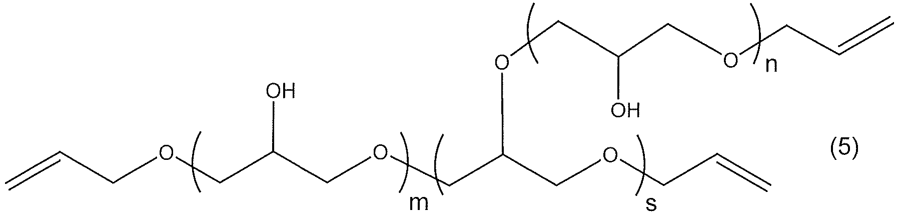

In the general formula (5), m is 1 or more, preferably 1 to 20, or more preferably 2 to 15; n is 1 or more, preferably 1 to 20, or more preferably 2 to 15; and s is 0 or more, preferably 0 to 10, or more preferably 0 to 5.

The method for producing the allyl-group-containing polyglycerin derivative (A5) is already known and is not particularly limited. For example, it may be produced by ring-opening and polymerizing glycidol in the presence of a catalyst and then reacting an allyl alcohol at a terminal of the polymer. The allyl-group-containing polyglycerin derivative (A5) may be used alone or in combination with a plurality of types.

(A2), (A3), (A4), and (A5) may be used alone or in combination with a plurality. The organosiloxane (A2) wherein e2 + f2 + g2 is 1 as well as the following (A6), (A7), and (A8) may be used in combination as needed. Note that the following (A6), (A7), and (A8) are regarded as modified groups of the hydrogen polysiloxane and are not comprised in the alkenyl-group-containing organic compounds for preparing the crosslinked organosiloxane polymer. The weight average molecular weight of (A2), (A3), (A4), and (AS) is preferably 50 to 9,000,000 or more preferably 50 to 900,000.

CH₂=CH-(CH₂)ᵢ-(OC₂H₄)j-(OCH(CH₃)CH₂)k-O-R³ (A6)

i, j and k are as defined above and R³ is an alkyl group having 1 to 30 carbon atoms or a hydrogen atom.

CH₂=CH-(CH₂)r-CH₃ (A7)

In the formula, 0 ≤ r. 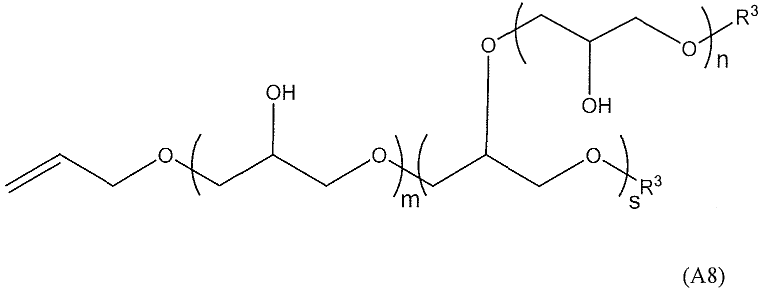 n, m and s are as defined above and R³ is an alkyl group having 1 to 30 carbon atoms or a hydrogen atom.

The reaction of the organohydrogen polysiloxane (A1) with the alkenyl-group-containing compound (A2), (A3), (A4), or (A5) is performed in the presence of a hydrosilylation reaction catalyst. The hydrosilylation reaction catalyst is not particularly limited, but examples thereof include platinum compounds such as chloroplatinic acid, an alcohol-modified chloroplatinic acid, and a complex of chloroplatinic acid and vinylsiloxane, and hydrosilylation reaction catalysts such as a rhodium compound. The organohydrogen polysiloxane (A1) may be reacted with the alkenyl-group-containing compounds (A2) to (AS) in the presence of a catalyst. In this case, the reaction temperature is not particularly limited but is preferably 40°C to 120°C. The amount of the hydrosilylation reaction catalyst to be added may be an effective amount. For example, the equivalent amount of platinum group metals is usually 0.1 ppm to 500 ppm, preferably 0.5 ppm to 200 ppm, or more preferably 1 ppm to 100 ppm, relative to the total amount.

In the hydrosilylation reaction, the molar ratio of (total alkenyl groups)/(total hydrosilyl groups) is not particularly limited but is preferably 1/10 to 10/1, more preferably 7/10 to 3/1, or even more preferably 8/10 to 2/1.

The hydrosilylation reaction may be performed without a solvent or in an organic solvent as needed. Examples of the organic solvents include: aliphatic alcohols such as methanol, ethanol, 2-propanol, and butanol; aromatic hydrocarbons such as benzene, toluene, and xylene; aliphatic or alicyclic hydrocarbons such as n-pentane, n-hexane, and cyclohexane; halogenated hydrocarbons such as dichloromethane, chloroform, and carbon tetrachloride; and ketone solvents such as acetone and methyl ethyl ketone. Among these, from the viewpoint of use for cosmetic applications, the reaction is preferably performed without a solvent, or using ethanol or 2-propanol. The organic solvents described above may be removed after the reaction by heating, reducing the pressure, and the like as needed. Part of the oil agent (B) described below may be added during the hydrosilylation reaction as needed.

### Oil agent (B)

One or more kinds of an oil agent (B) that is liquid at 25°C are preferably blended into the cosmetic of the present invention. The amount of the oil agent (B) to be blended into the cosmetic may be a known blending amount depending on the type and the like of the cosmetic. The crosslinked organosiloxane polymer is preferably mixed with part of the oil agent (B) in advance to form a homogeneous organosiloxane mixture. At this time, it is more preferably a liquid, paste, or gel. Since the crosslinked organosiloxane polymer is non-fluid, it is easily blended directly into the cosmetic by mixing it with part of the oil agent (B) in advance. In addition, homogeneously dispersing the crosslinked organosiloxane polymer in the cosmetic is preferable because it provides a good appearance and a good feeling when used as well as excellent stability.

The oil agent (B) is preferably a liquid oil with a kinematic viscosity of 0.65 to 10,000 mm²/s at 25°C in terms of ease of handling. Note that, in the present invention, the kinematic viscosity is measured by the method using a Cannon-Fenske viscometer described in the Japanese Industrial Standards (JIS) Z 8803:2011. The type of oil agent is not particularly limited. Examples of preferable oil agents include the silicone oils, hydrocarbon oils, fluorinated oils, ester oils, natural animal and vegetable oils, and semi-synthetic oils described below.

Examples of the silicone oils include: alkyl-modified silicones such as dimethicone (INCI), cyclopentasiloxane (INCI), cyclohexasiloxane (INCI), ethyl methicone (INCI), ethyl trisiloxane (INCI), and hexyl dimethicone (INCI); long-chain alkyl-modified silicones such as caprylyl methicone (INCI); straight-chain or branched organopolysiloxanes such as phenyl trimethicone (INCI), diphenylsiloxy phenyl trimethicone (INCI), diphenyl dimethicone (INCI), and tetraphenyl dimethyl disiloxane (INCI); aromatic-group-modified silicones such as tetramethyl tetraphenyl cyclotetrasiloxane and other cyclic organopolysiloxanes; amino-modified organopolysiloxanes such as amodimethicone (INCI) and aminopropyl dimethicone (INCI); pyrrolidone-modified organopolysiloxanes such as PCA dimethicone (INCI); PCA-modified organopolysiloxanes; amino-acid-modified silicones; and fluorine-modified silicones.

Examples of the hydrocarbon oils include chain and cyclic hydrocarbon oils. Examples thereof include: olefin oligomers (INCI); isoparaffins such as C13-14 isoparaffin (INCI); isododecane (INCI); undecane (INCI); dodecane (INCI); isohexadecane (INCI); hydrogenated polyisobutene (INCI); squalane (INCI); mineral oil (INCI); and alkanes such as coconut alkanes (INCI) and C13-15 alkane (INCI).

Examples of the fluorinated oils include perfluoropolyethers, perfluorodecalin, and perfluorooctane.

Examples of the ester oils include: diisobutyl adipate (INCI); dihexyldecyl adipate (INCI); diheptylundecyl adipate (INCI); n-alkyl glycol mono isostearates such as isostearyl isostearate (INCI); isocetyl isostearate (INCI); trimethylolpropane triisostearate (INCI); glycol diethylhexanoate (INCI); cetyl ethylhexanoate (INCI); trimethylolpropane triethylhexanoate (INCI); pentaerythrityl tetraethylhexanoate (INCI); octyldodecyl esters such as octyldodecyl stearoyl stearate (INCI); oleyl oleate (INCI); octyldodecyl oleate (INCI); decyl oleate (INCI); neopentyl glycol diethylhexanoate (INCI); neopentyl glycol dicaprate (INCI); diisostearyl malate (INCI); triethyl citrate (INCI); diethylhexyl succinate (INCI); amyl acetate (INCI); ethyl acetate (INCI); butyl acetate (INCI); isocetyl stearate (INCI); butyl stearate (INCI); diisopropyl sebacate (INCI); diethylhexyl sebacate (INCI); cetyl lactate (INCI); myristyl lactate (INCI); isononyl isononanoate (INCI); isotridecyl isononanoate (INCI); palmitates such as isopropyl palmitate (INCI), ethylhexyl isopalmitate (INCI), and isocetyl palmitate or hexyldecyl palmitate (INCI); cholesteryl hydroxystearate (INCI); myristates such as isopropyl myristate (INCI), octyldodecyl myristate (INCI), and myristyl myristate (INCI); ethylhexyl laurate (INCI); hexyl laurate (INCI); dioctyldodecyl lauroyl glutamate (INCI); and isopropyl lauroyl sarcosinate (INCI).

Among the ester oils, examples of glyceride oils include triethylhexanoin (INCI), caprylic/capric triglyceride (INCI), cocoglycerides (INCI), caprylic/capric/succinic triglyceride (INCI), and caprylic/capric glycerides (INCI).

Examples of the natural animal and vegetable oil agents and semi-synthetic oil agents include: germ oils such as Persea gratissima (avocado) oil (INCI), Linum usitatissimum (linseed) seed oil (INCI), Prunus amygdalus dulcis (sweet almond) oil (INCI), Perilla frutescens seed oil (INCI), Olea europaea (olive) fruit oil (INCI), Torreya californica (California nutmeg) oil (INCI), Cymbopogon nardus (citronella) oil (INCI), Torreya nucifera seed oil (INCI), kyounin yu (INCI), Triticum vulgare (wheat) germ oil (INCI), Sesamum indicum (sesame) seed oil (INCI), Triticum vulgare (wheat) germ oil (INCI), Oryza sativa (rice) germ oil (INCI), Oryza sativa (rice) bran oil (INCI), Camellia kissi seed oil (INCI), Carthamus tinctorius (safflower) seed oil (INCI), Glycine soja (soybean) oil (INCI), Camellia sinensis seed oil (INCI), Camellia japonica seed oil (INCI), Oenothera biennis (evening primrose) oil (INCI), rape shushi yu (INCI), and Zea mays (corn) germ oil (INCI); natural vegetable oils such as persic oil (INCI), Elaeis guineensis (palm) oil (INCI), Elaeis guineensis (palm) kernel oil (INCI), Ricinus communis (castor) seed oil (INCI), Helianthus annuus (sunflower) seed oil (INCI), Vitis vinifera (grape) seed oil (INCI), Simmondsia chinensis (jojoba) seed oil (INCI), Macadamia ternifolia seed oil (INCI), Limnanthes alba (meadowfoam) seed oil (INCI), Gossypium herbaceum (cotton) seed oil (INCI), Cocos nucifera (coconut) oil (INCI), and Arachis hypogaea (peanut) oil (INCI); natural animal oils such as shark liver oil (INCI), cod liver oil (INCI), fish liver oil (INCI), turtle oil (INCI), mink oil (INCI), and egg oil (INCI); and semi-synthetic oils and fats such as hydrogenated coconut oil (INCI) and lanolin oil (INCI).

Among them, the oil agent (B) is preferably a modified silicone oil or an ester oil. As the modified silicone oil, dimethicone (INCI), ethyl methicone (INCI), silicone oils that have a phenyl group such as phenyl trimethicone (INCI), diphenylsiloxy phenyl trimethicone (INCI), and diphenyl dimethicone (INCI), and straight-chain or branched organopolysiloxanes such as a dimethylsiloxane-methylphenylsiloxane copolymer are particularly preferable. Examples of commercially available products include dimethicone (examples thereof include KF-96A-6cs, KF-96L-2cs, and KF-995, manufactured by Shin-Etsu Chemical Co., Ltd.) and diphenylsiloxy phenyl trimethicone (KP-56A, manufactured by Shin-Etsu Chemical Co., Ltd.).

As the ester oil, triethylhexanoin (INCI), isononyl isononanoate (INCI), isotridecyl isononanoate (INCI), cetyl ethylhexanoate (INCI), caprylic/capric triglyceride (INCI), and ethylhexyl isopalmitate (INCI) are particularly preferable.

The mixing ratio of the crosslinked organosiloxane polymer (A) and the oil agent (B) is not particularly limited. For example, (crosslinked organosiloxane polymer)/(oil agent (B)) is preferably 1/30 to 30/1 (mass ratio) or more preferably 1/20 to 10/1 (mass ratio). The method for mixing is not limited and may be a known method using, for example, a three-roll mill, a two-roll mill, a kneader, a mass colloider, a sand grinder, a colloid mill, a Gaulin Homogenizer, a disper, or a high-shear mixer. Methods using a three-roll mill or a disper are preferable to obtain a paste composition with a smooth appearance.

### <Other components>

The various components described below that may be blended into the cosmetic maybe blended into the cosmetic of the present invention as other components. These may be used alone or in an appropriate combination of two or more kinds. The blending amount of the other components may be appropriately selected depending on factors such as the type of cosmetic and may be a known blending amount depending on factors such as the type of cosmetic.

### • UV absorbers

The UV absorbers are not particularly limited and are only required to be UV absorbers that may be blended into conventional cosmetics. Examples thereof include homosalate (INCI), octocrylene (INCI), butyl methoxydibenzoylmethane (INCI), ethylhexyl salicylate (INCI), diethylamino hydroxybenzoyl hexyl benzoate (INCI), benzophenone-6 (INCI), benzophenone-9 (INCI), benzophenone-1 (INCI), polysilicone-15 (INCI), ethylhexyl dimethoxybenzylidene dioxoimidazolidine propionate (INCI), benzophenone-2 (INCI), terephthalylidene dicamphor sulfonic acid (INCI), ethylhexyl triazone (INCI), isopentyl trimethoxycinnamate trisiloxane (INCI), drometrizole trisiloxane (INCI), ethylhexyl dimethyl PABA (INCI), isopropyl methoxycinnamate (INCI), ethylhexyl methoxycinnamate (INCI), bis-ethylhexyloxyphenol methoxyphenyl triazine (INCI), benzophenone-3 (INCI), benzophenone-4 (INCI), benzophenone-5 (INCI), phenylbenzimidazole sulfonic acid (INCI), methylene bisbenzotriazolyl tetramethylbutylphenol (INCI), glyceryl ethylhexanoate dimethoxycinnamate (INCI), glyceryl PABA (INCI), diisopropyl methyl cinnamate (INCI), cinoxate (INCI), and ethylhexyl dimethoxybenzylidene dioxoimidazolidine propionate (INCI).

UVA absorbers (examples thereof include diethylamino hydroxybenzoyl hexyl benzoate (INCI)) and UVB absorbers (examples thereof include ethylhexyl methoxycinnamate (INCI)) may be used in combination. Each of them may be optionally combined. The blending amount of the UV absorbers is not particularly limited but is preferably 0.1% to 35% by mass, more preferably 5% to 27% by mass, even more preferably 7% to 20% by mass, or most preferably 10% to 20% by mass, relative to the entire cosmetic.

### • Aqueous components

The aqueous components are not particularly limited and are only required to be aqueous components that may be blended into conventional cosmetics. Examples thereof include water; lower alcohols such as ethanol; sugar alcohols such as erythritol, maltitol, xylitol, and sorbitol; and moisturizers such as 1,3-butylene glycol (1,3-BG), glycerin, propylene glycol (PG), dipropylene glycol (DPG), pentylene glycol, and other polyhydric alcohols. These may be used alone or in an appropriate combination of two or more kinds. The blending amount of the aqueous components in the cosmetic is preferably 0.1% to 90% by mass.

### • Oily components other than Component (B)

The oily components other than Component (B) are not particularly limited and are only required to be oily components used in conventional cosmetics. Examples thereof include esters, petrolatum, and lanolin that are pastes at 25°C, as well as waxes and silicone waxes that are solid.

### • Powders

The powders are not particularly limited and are only required to be ingredients that may be blended into conventional cosmetics. Examples thereof include color pigments, inorganic powders, organic powders, inorganic-organic composite powders, and metal powders. The blending amount of the powder is not particularly limited but is preferably 0.1% to 90% by mass, more preferably 1% to 35% by mass, or even more preferably 1% to 18% by mass, relative to the entire cosmetic.

The color pigments may be any of the following: red iron oxide; yellow iron oxide; white titanium dioxide; black iron oxide; red ocher; ultramarines; ferric ferrocyanide; manganese violet; cobalt violet; chromium hydroxide green; chromium oxide greens; cobalt oxide; cobalt titanium oxide; iron-oxide-doped titanium dioxide; iron titanium oxide; titanium/titanium dioxide; lithium cobalt titanate; inorganic brown pigments such as titanium nitride, iron hydroxide, and γ-iron oxide; inorganic yellow pigments such as yellow ocher; and colored pigments such as tar-based lake pigments and natural lake pigments.

Examples of the inorganic powders include fine particles made of zirconium dioxide, zinc oxide, cerium oxide, magnesium oxide, barium sulfate, calcium sulfate, magnesium sulfate, calcium carbonate, magnesium carbonate, talc, cleaved talc, mica, kaolin, sericite, muscovite, synthetic fluorphlogopite, fluorphlogopite, lepidolite, biotite, lithia mica, silicic acid, silica, fumed silica, hydrated silica, aluminum silicate, magnesium silicate, magnesium aluminum silicate, calcium silicate, barium silicate, strontium silicate, metal salts of tungstic acid, hydroxyapatite, vermiculite, higilite, bentonite, montmorillonite, hectorite, zeolite, ceramics, dicalcium phosphate, alumina, aluminum hydroxide, boron nitride, boron nitride and glass. Examples of inorganic color pearl pigments include pearl pigments such as titanium-dioxide-coated mica, bismuth oxychloride, titanium-dioxide-coated bismuth oxychloride, titanium-dioxide-coated talc, fish scale flakes, and titanium-dioxide-coated colored mica. Examples of inorganic powders for UV-shielding applications include titanium dioxide fine particles, zinc oxide fine particles, and cerium oxide fine particles, and titanium dioxide fine particles and zinc oxide fine particles are preferable in terms of UV shielding and usability. These inorganic powders for UV shielding may be used alone or in combination and are appropriately selected based on their absorption wavelengths and dispersibility.

The blending amount of the inorganic powder for UV-shielding applications is not particularly limited but is preferably 0.1% to 35% by mass, more preferably 1% to 18% by mass, even more preferably 1% to 10% by mass, or most preferably 1% to 5% by mass, relative to the entire cosmetic.

Examples of the organic powders include powders made of silicone, polyamide, polyacrylates/acrylates, polyester, polyethylene, polypropylene, polystyrene, styrene/acrylates copolymers, styrene/divinylbenzene copolymers, polyurethane, vinyl resins, urea resins, melamine resins, benzoguanamine, polymethyl benzoguanamine, tetrafluoroethylene, polymethyl methacrylates (examples thereof include polymethyl methacrylate), cellulose, silk, nylon, phenolic resins, epoxy resins, or polycarbonates. In particular, examples of silicones include silicone resin particles (examples thereof include KMP-590, KMP-591, and KMP-592, manufactured by Shin-Etsu Chemical Co., Ltd.), silicone rubber powders (examples thereof include KMP-597 and KMP-598, manufactured by Shin-Etsu Chemical Co., Ltd.), silicone-resin-coated silicone rubber powders (examples thereof include KSP-100, KSP-101, KSP-102, KSP-105, KSP-300, KSP-411, and KSP-441, manufactured by Shin-Etsu Chemical Co., Ltd.), and they may be dispersed in water or oil in advance (examples thereof include KM-9729 and KSG-016F, manufactured by Shin-Etsu Chemical Co., Ltd.). Metallic soaps and the like are also included, and examples thereof include powders made of zinc stearate, aluminum stearate, calcium stearate, magnesium stearate, zinc myristate, magnesium myristate, zinc cetyl phosphate, calcium cetyl phosphate, and sodium zinc cetyl phosphate. In addition, organic colors and the like are also included, and examples thereof include tar colors such as Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 505, Yellow No. 4, Yellow No. 5, Yellow No. 202, Yellow No. 203, Yellow No. 204, Yellow No. 401, Blue No. 1, Blue No. 2, Blue No. 201, Blue No. 404, Green No. 3, Green No. 201, Green No. 204, Green No. 205, Orange No. 201, Orange No. 203, Orange No. 204, Orange No. 206, and Orange No. 207 as well as natural colors such as carminic acid, laccaic acid, carthamin, brazilin, and crocin.

Examples of the inorganic-organic composite powders include composite powders wherein the surface of the inorganic powder is coated with organic powder by means of a known and accepted method.

Examples of the metal powders include metal fine particles made of aluminum, copper, stainless steel, and silver.

The powders described above may be powders whose particle surfaces are treated. Surface treatment agents are preferably those that impart hydrophobicity from the viewpoint of water resistance of the cosmetic. The treatment agents that impart hydrophobicity are not particularly limited, and examples thereof include: silicone treatment agents; waxes; paraffins; organic fluorine compounds made of perfluoroalkyls and phosphates; surfactants; amino acids such as N-acylglutamic acid; and metallic soaps such as aluminum stearate and magnesium myristate.

The silicone treatment agents are more preferably: silanes or silylating agents such as caprylylsilanes (AES-3083, manufactured by Shin-Etsu Chemical Co., Ltd.) or trimethoxysilyl dimethicone; silicone oils such as dimethyl silicones (KF-96A series, manufactured by Shin-Etsu Chemical Co., Ltd.), methicones (KF-99P and KF-9901, manufactured by Shin-Etsu Chemical Co., Ltd.), and silicone-branched silicone treatment agents (KF-9908 and KF-9909, manufactured by Shin-Etsu Chemical Co., Ltd.); or silicone acrylates (KP-574 and KP-541, manufactured by Shin-Etsu Chemical Co., Ltd.). Furthermore, the surface hydrophobization treatment agents may be used alone or in combination of two or more kinds.

Among the silicone treatment agents, triethoxysilylethyl polydimethylsiloxyethyl hexyl dimethicone (KF-9909, manufactured by Shin-Etsu Chemical Co., Ltd.), which is a dimethicone that has a triethoxysilyl group, a polydimethylsiloxyethyl group, and a hexyl group in the side chain and the like are preferable. The silicone treatment agents may demonstrate high affinity even when the dispersing medium for dispersing color pigments that have been highly hydrophobized is a mixture of silicone and hydrocarbons or the like. Furthermore, the surface hydrophobization treatment agents may be used alone or in combination of two or more kinds. Examples of the color pigments whose surface has been hydrophobized include the KTP-09 series, in particular, KTP-09W, KTP-09R, KTP-09Y, and KTP-09B (manufactured by Shin-Etsu Chemical Co., Ltd.).

### • Surfactants

The surfactants include nonionic, anionic, cationic, and amphoteric surface-active agents but are not particularly limited and are only required to be surfactants used in conventional cosmetics. Among these surfactants, crosslinked polyether-modified silicones, crosslinked polyglycerin-modified silicones, straight-chain or branched polyoxyethylene-modified organopolysiloxanes, straight-chain or branched polyoxyethylene-polyoxypropylene-modified organopolysiloxanes, straight-chain or branched polyoxyethylene- and alkyl-co-modified organopolysiloxanes, straight-chain or branched polyoxyethylene-polyoxypropylene- and alkyl-co-modified organopolysiloxanes, straight-chain or branched polyglycerin-modified organopolysiloxanes, straight-chain or branched polyglycerin- and alkyl-co-modified organopolysiloxanes, and straight-chain or branched pyrrolidone-modified organopolysiloxanes having a structure different from Component (A) are preferable.

In the surfactants, the amount of contained hydrophilic polyoxyethylene groups, polyoxyethylene-polyoxypropylene groups, or polyglycerin residues is preferably 10% to 70% by mass in the molecule.

When a crosslinked organopolysiloxane such as crosslinked polyether-modified silicone or crosslinked polyglycerin-modified silicone is used, in a composition made of the crosslinked organopolysiloxane and an oil agent that is liquid at room temperature, the crosslinked organopolysiloxane is preferably swollen with the liquid oil using an amount equal to or more than its own weight. As the liquid oil agent, liquid silicones, hydrocarbon oils, ester oils, natural animal and vegetable oils, semi-synthetic oils, and the like, or fluorinated oils in the oil agent of Component (B) may be used, and examples thereof include silicones with a low viscosity of 0.65 to 100 mm²/s (at 25°C), hydrocarbon oils such as mineral oil, squalane, isododecane, and isohexadecane, glyceride oils such as triethylhexanoin, ester oils such as isotridecyl isononanoate, N-acylglutamate, and lauroyl sarcosinate, and natural animal and vegetable oils such as Macadamia ternifolia seed oil.

Examples of the crosslinked organopolysiloxanes swollen with the liquid oil agent include KSG-210, KSG-240, KSG-310, KSG-320, KSG-330, KSG-340, KSG-320Z, KSG-350Z, KSG-710, KSG-810, KSG-820, KSG-830, KSG-840, KSG-820Z, and KSG-850Z, manufactured by Shin-Etsu Chemical Co., Ltd.

Examples of the surfactants that are not a crosslinked organopolysiloxane include KF-6011, KF-6013, KF-6043, KF-6017, KF-6028, KF-6038, KF-6048, KF-6100, KF-6104, KF-6105, and KF-6106, manufactured by Shin-Etsu Chemical Co., Ltd.

The blending amount of the surfactants is preferably 0.1% to 20% by mass, relative to the entire cosmetic. An amount of 0.1% by mass or more is preferable because the dispersion and emulsification functions are sufficiently fulfilled, while an amount of 20% by mass or less is preferable because the cosmetic does not risk having a sticky feeling when used. The HLB of the surfactants is not limited but is preferably 2 to 14.5 for the purpose of maintaining the water resistance of the cosmetic.

### • Crosslinked organopolysiloxanes

One or more types of crosslinked organopolysiloxanes used in conventional cosmetics that are different from Component (A) maybe used alone or in an appropriate combination, as long as the effects of the present invention are not impaired. The crosslinked organopolysiloxanes do not have a spherical shape, unlike the powders above. They are compounds that do not have a polyether or polyglycerin structure in their molecular structure, unlike the surfactants. They are elastomers that have structural viscosity when swollen with Component (B).

Examples thereof include dimethicone/vinyl dimethicone crosspolymer, dimethicone/phenyl vinyl dimethicone crosspolymer, vinyl dimethicone/lauryl dimethicone crosspolymer, and lauryl polydimethylsiloxyethyl dimethicone/bis-vinyl dimethicone crosspolymer, as defined in the International Nomenclature of Cosmetic Ingredients (INCI). They are commercially available as swollen products including an oil that is liquid at room temperature. Examples thereof include KSG-15, KSG-1510, KSG-16, KSG-1610, KSG-18A, KSG-19, KSG-41A, KSG-42A, KSG-43, KSG-44, KSG-042Z, KSG-045Z, and KSG-048Z, manufactured by Shin-Etsu Chemical Co., Ltd. The blending amount of the crosslinked organopolysiloxane is preferably 0.01% to 30% by mass in the cosmetic in terms of solids content.

### • Coating agents

The coating agents are not particularly limited and are only required to be ingredients that may be blended into conventional cosmetics. For example, polyvinyl alcohol, PVP, latexes such as polyvinyl acetate and polyalkylacrylate, dextrins, cellulose derivatives such as alkylcellulose and nitrocellulose, silicone-modified polysaccharide compounds such as trimethylsiloxysilylcarbamoyl pullulan, silicone acrylate graft copolymers such as an acrylates/dimethicone copolymer, silicone resins such as trimethylsiloxysilicate, silicone-based resins such as silicone-modified polynorbornene and a fluorine-modified silicone resin, fluoropolymers, aromatic hydrocarbon resins, polymer emulsion resins, terpene-based resins, polybutene, polyisoprene, alkyd resins, PVP-modified polymers, rosin-modified resins, and polyurethane are used.

Among these, silicone-based coating agents are particularly preferable. Among them, trimethylsiloxysilylcarbamoyl pullulan (examples of a commercially available product that is dissolved in a solvent include TSPL-30-D5 and TSPL-30-ID, manufactured by Shin-Etsu Chemical Co. Ltd.), acrylates/dimethicone copolymers (examples of a commercially available product that is dissolved in a solvent include KP-543, KP-545, KP-549, KP-550, and KP-545L, manufactured by Shin-Etsu Chemical Co., Ltd.), trimethylsiloxysilicate (examples of a commercially available product that is dissolved in a solvent include KF-7312J and X-21-5250, manufactured by Shin-Etsu Chemical Co., Ltd.), silicone-modified polynorbornene (examples of a commercially available product that is dissolved in a solvent include NBN-30-ID, manufactured by Shin-Etsu Chemical Co., Ltd.), and organosiloxane graft polyvinyl alcohol polymers may be suitably used. They are not limited to these. The coating agents may be used alone or in combination with two or more kinds. The blending amount of the coating agents in the cosmetic is preferably 0.1% to 20% by mass.

### • Other additives

Examples of the other additives include oil-soluble gelling agents, water-soluble thickening agents, antiperspirants, preservatives/antiseptics, fragrances, salts, antioxidants, pH adjusters, chelating agents, refreshing agents, anti-inflammatory agents, skin-beautifying components (examples thereof include skin-lightening agents, cell activators, rough-skin-improving agents, blood circulation promoters, skin astringents, and anti-seborrheics), vitamins, amino acids, nucleic acids, hormones, and inclusion compounds.

### • Oil-soluble gelling agents

Examples of the oil-soluble gelling agents include: metallic soaps such as aluminum stearate, magnesium stearate, and zinc myristate; amino acid derivatives such as N-lauroyl-L-glutamic acid and α, γ-di-n-butylamine; dextrin fatty acid esters such as dextrin palmitate, dextrin stearate, and dextrin palmitate/ethylhexanoate; sucrose fatty acid esters such as sucrose palmitate and sucrose stearate; fructooligosaccharide fatty acid esters such as fructooligosaccharide stearate and fructooligosaccharide 2-ethylhexanoate; benzylidene derivatives of sorbitol such as monobenzylidene sorbitol and dibenzylidene sorbitol; and organically modified clay minerals of disteardimonium hectorite, stearalkonium hectorite, and hectorite.

### • Water-soluble thickening agents

Examples of the water-soluble thickening agents include: plant-based polymers such as Acacia senegal gum, Astragalus gummifer gum, galactan, Ceratonia siliqua (carob) gum, Cyamopsis tetragonoloba (guar) gum, Sterculia urens gum, carrageenan, pectin, agar, Pyrus cydonia seed (Cydonia oblonga), starches (examples thereof include Oryza sativa (rice) starch, Zea mays (corn) starch, Solanum tuberosum (potato) starch, and Triticum vulgare (wheat) starch), and algin; microbial polymers such as xanthan gum, dextran, succinoglucan, and pullulan; animal-based polymers such as collagen, casein, albumin, and gelatin; starch-based polymers such as carboxymethyl starch and methyl hydroxypropyl starch; cellulose-based polymers such as methylcellulose, ethylcellulose, hydroxypropyl methylcellulose, carboxymethyl cellulose, hydroxymethylcellulose, hydroxypropylcellulose, nitrocellulose, sodium cellulose sulfate, sodium carboxymethylcellulose, microcrystalline cellulose, cationic cellulose, and cellulose powder; alginate-based polymers such as algin and propylene glycol alginate; vinyl polymers such as polyvinyl methyl ether and a carboxyvinyl polymer; polyoxyethylene polymers; polyoxyethylene-polyoxypropylene-copolymer-based polymers; acrylic polymers such as sodium polyacrylate, polyethyl acrylate, polyacrylamide, and an acryloyldimethyl taurate copolymer; other synthetic water-soluble polymers such as PEI and a cationic polymer; and inorganic water-soluble polymers such as bentonite, magnesium aluminum silicate, montmorillonite, beidellite, nontronite, saponite, hectorite, and silicic anhydride.

Among these, one or more kinds of water-soluble thickening agents selected from plant-based polymers, microbial polymers, animal-based polymers, starch-based polymers, cellulose-based polymers, alginate-based polymers, polyoxyethylene-polyoxypropylene-copolymer-based polymers, acrylic polymers, and inorganic water-soluble polymers are preferably used.

### • Antiperspirants

Examples of the antiperspirants include: aluminum hydroxy-halides such as aluminum chlorohydrate and alcloxa; aluminum halides such as aluminum chloride; aluminum salts of allantoin; tannic acid; persimmon tannin; potassium alum; zinc oxide; zinc p-phenolsulfonate; burnt alum; aluminum zirconium tetrachlorohydrate; and aluminum zirconium trichlorohydrex GLY. In particular, as components that demonstrate strong effects, aluminum hydroxy-halides, aluminum halides, their complexes or mixtures with zirconyl oxy-halides and zirconyl hydroxy-halides (examples thereof include aluminum zirconium tetrachlorohydrate and aluminum zirconium trichlorohydrex GLY), and the like are preferable.

### • Preservatives/antiseptics

Examples of the preservatives/antiseptics include parabens, benzoic acid, sodium benzoate, sorbic acid, potassium sorbate, phenoxyethanol, imidazolidinyl urea, salicylic acid, o-cymen-5-ol, phenol, p-chloro-m-cresol, hexachlorophene, benzalkonium chloride, chlorhexidine chloride, trichlorocarbanilide, iodopropynyl butylcarbamate, polylysine, photosensitizers, silver, and plant extracts.

### • Fragrances

Examples of the fragrances include natural fragrances and synthetic fragrances. Examples of the natural fragrances include: plant-based fragrances isolated from flowers, leaves, wood, and peels; and animal-based fragrances such as musk and civet. Examples of the synthetic fragrances include: hydrocarbons such as monoterpenes; alcohols such as aliphatic alcohols and aromatic alcohols; aldehydes such as terpene aldehydes and aromatic aldehydes; ketones such as alicyclic ketones; esters such as terpene-based esters; lactones; phenols; oxides; nitrogencontaining compounds; and acetals.

### • Salts

Examples of the salts include inorganic salts, organic acid salts, amine salts, and amino acid salts. Examples of the inorganic salts include sodium salts, potassium salts, magnesium salts, calcium salts, aluminum salts, zirconium salts, and zinc salts of inorganic acids such as hydrochloric acid, sulfuric acid, carbonic acid, and nitric acid. Examples of the organic acid salts include salts of organic acids such as acetic acid, dehydroacetic acid, citric acid, malic acid, succinic acid, ascorbic acid, and stearic acid. Examples of the amine salts and amino acid salts include salts of amines such as triethanolamine and salts of amino acids such as glutamic acid. Other examples include salts of hyaluronic acid and chondroitin sulfate, as well as neutral salts of acids and alkalis used in drug formulations.

### • Antioxidants

Examples of the antioxidants include carotenoids, ascorbic acid and its salts, ascorbyl stearate, tocophenols, tocopheryl acetate, tocopherols, 4-tert-butylphenol, BHA, BHT, phytic acid, ferulic acid, thiotaurine, aminoethanesulfinic acid, sulfite salts, erythorbic acid and its salts, chlorogenic acids, epicatechin, epigallocatechin, epigallocatechin gallate, apigenin, kaempferol, myricetin, and quercetin.

### • pH adjusters

Examples of the pH adjusters include lactic acid, citric acid, glycolic acid, succinic acid, tartaric acid, DL-malic acid, potassium carbonate, sodium bicarbonate, and ammonium bicarbonate.

### • Chelating agents

Examples of the chelating agents include alanine, sodium salts of EDTA acid, sodium polyphosphate, sodium metaphosphate, and phosphoric acid.

### • Refreshing agents

Examples of the refreshing agents include L-menthol, camphor, and menthyl lactate.

### • Anti-inflammatory agents

Examples of the anti-inflammatory agents include allantoin, glycyrrhizic acid and its salts, glycyrrhetinic acid, stearyl glycyrrhetinate, tranexamic acid, and azulene.

### • Skin-beautifying components

Examples of the skin-beautifying components include: skin-lightening agents such as placental extracts, arbutin, glutathione, tranexamic acid, ascorbic acid derivatives, and Saxifraga sarmentosa extracts; cell activators such as royal jelly, photosensitizers, cholesterol derivatives, and calf blood extracts; rough-skin-improving agents; blood circulation promoters such as hydroxymethoxybenzyl pelargonamide, benzyl nicotinate, β-butoxyethyl nicotinate, capsaicin, zingerone, cantharides tincture, ichthammol, caffeine, tannic acid, α-borneol, tocopheryl nicotinate, inositol hexaniacinate, cyclandelate, cinnarizine, tolazoline, acetylcholine, verapamil, cepharanthine, and γ-oryzanol; skin astringents; and anti-seborrheics such as sulfur and thianthol.

### • Vitamins

Examples of the vitamins include: vitamin A, such as vitamin A oils, retinol, retinyl acetate, and retinyl palmitate; vitamin B2, such as riboflavin, riboflavin butyrate, and flavin adenine dinucleotide; vitamin B6, such as pyridoxine HCl, pyridoxine dicaprylate, and pyridoxine tripalmitate; vitamin B, such as vitamin B12, vitamin B15, and their derivatives; vitamin C, such as L-ascorbic acid, L-ascorbyl dipalmitate, disodium L-ascorbyl sulfate, and dipotassium L-ascorbyl phosphate; vitamin D, such as ergocalciferol and cholecalciferol; vitamin E, such as α-tocopherol, β-tocopherol, γ-tocopherol, DL-α-tocopheryl acetate, DL-α-tocopheryl nicotinate, and DL-α-tocopheryl succinate; niacin, such as nicotinic acid, benzyl nicotinate, and niacinamide; vitamin H; vitamin P; pantothenic acids such as calcium pantothenate, D-panthenol, panthenyl ethyl ether, and panthenyl ethyl ether acetate; and biotin.

### • Amino acids

Examples of the amino acids include glycine, valine, leucine, isoleucine, serine, threonine, phenylalanine, arginine, lysine, aspartic acid, glutamic acid, cystine, cysteine, methionine, and tryptophan.

### • Nucleic acids

Examples of the nucleic acids include deoxyribonucleic acid.

### • Hormones

Examples of the hormones include estradiol and ethenylestradiol.

### • Inclusion compounds

Examples of the inclusion compounds include cyclodextrin.

### <Cosmetic>

The crosslinked organosiloxane polymer of the present invention may be blended into cosmetics for various applications. It is particularly applicable as an ingredient for all cosmetics used externally on the skin and hair. The amount of the crosslinked organosiloxane polymer to be blended into the cosmetic is not particularly limited but may be appropriately adjusted depending on the type and the purpose of the cosmetic. For example, it is preferably within the range of 0.01% to 95% by mass, more preferably 0.05% to 80% by mass, or even more preferably 0.1% to 50% by mass, relative to the entire cosmetic. Blending in the crosslinked organosiloxane polymer in such an amount provides a cosmetic that has a good feeling when used.

The form of the cosmetic may be emulsion-based, non-aqueous, or aqueous. An emulsion-based form is preferable to impart a fresh feeling when used. The emulsion-based form may be an O/W emulsion, a W/O emulsion, an O/W/O emulsion, or a W/O/W emulsion. A non-aqueous composition or a powder composition is preferable to obtain an oily feeling or water resistance. An aqueous composition is preferable to obtain a refreshing feeling when used. The present invention provides a good cosmetic in any of the forms. Note that in the present invention, "non-aqueous composition" refers to a composition wherein water is not intentionally blended therein, and "aqueous composition" refers to a composition wherein oily components are not intentionally blended therein. Among these, W/O emulsions and non-aqueous compositions, which are expected to have strong thickening properties, are particularly preferable. Examples of the W/O emulsions include a two-layer separated type that requires it to be shaken well before use and a non-separated type that is used as is without shaking.

The two-layer separated type, which comes in several varieties, refers in a broad sense to one in which an emulsion composition or a dispersion separates into multiple phases when left to stand. For example, when left to stand, some separate into two layers with a powder-including layer and an emulsion layer, some separate into two layers with a powder-including layer and an oil layer, some separate into two layers with a water layer and an oil layer, some are partially separated into an emulsion layer and a water layer, some are partially separated into a powder-including emulsion layer and a water layer, and some are separated into a layer made of powder and oil-layer components and a water layer. All are characterized by having a single emulsified and dispersed or dispersed layer immediately after shaking. Some formulations have a stainless steel ball inside a container to easily mix the ingredients when shaking. These two-layer separated types include a two-layer type, a shaking type, and a rattling shaking type. The W/O emulsion preferably demonstrates the effects of the present invention in a two-layer separated type that requires it to be shaken well before use or in a non-separated type that may be used as is without shaking. The non-separated type, which does not require shaking, is preferable in terms of usability.

The cosmetic including the crosslinked organosiloxane polymer of the present invention may be prepared by various known methods. It may also be mixed with the oil agents, the UV absorbers, and the powders described above. In the case of an emulsion composition, it may be mixed with the oil phase before emulsification, with the emulsion after emulsification, or with both. Mixing may be performed at room temperature or under heating. Methods for mixing are not particularly limited, and may use a pulsator, a propeller mixer, a paddle mixer, a disper, a homomixer, a roll, a Henschel-Mixer, an atomizer, a pin mill, a rotation-revolution mixer, or the like.

The cosmetic is not particularly limited, but the present invention may be applied to various products such as serums, milky lotions, creams, hair care products, foundations, makeup bases, sunscreens, concealers, blushes, lipsticks, lip glosses, balms, mascaras, eyeshadows, eyeliners, body makeup products, deodorants, and nail cosmetics. Among these, makeup cosmetics such as milky lotions, creams, hair care products, and foundations and cosmetics that impart sunscreen effects are particularly preferable. The physical form of the cosmetic of the present invention may be selected from various forms such as a liquid form, a cream form, a solid form, a paste form, a gel form, a mousse form, a soufflé form, a clay form, a powder form, and a stick form.

### EXAMPLES

The present invention will be explained below in further detail with reference to a series of the Examples and the Comparative Examples, though the present invention is in no way limited by these Examples.

Note that the solid-or-liquid determination and the measurement of the loss coefficient described in the examples and the comparative examples were performed as follows.

### • Solid-or-liquid determination

Mark lines at 25 mm and 40 mm from the bottom of a 20 mL screw tube (LABORAN Screw Tube Bottle No. 5, manufactured by AS ONE CORPORATION) and inject a crosslinked organosiloxane polymer up to the line marked at 25 mm. Next, seal the sample and leave it to stand at a sustained temperature in a thermostatic chamber at 25°C for 24 hours. Then, tilt the screw tube horizontally. The sample is determined to be "a liquid" if the tip of the sample reaches the line marked at 40 mm within 96 hours or "a solid" if the tip of the sample does not reach the line.

### • Loss coefficient

Measurements were performed using a Modular Compact Rheometer MCR102 (manufactured by Anton Paar). Measurements were performed using a 25 mm parallel plate at a measurement temperature of 25°C with a gap of 0.5 mm and strain of 10%, with the measured values at shear frequencies of 1 Hz and 10 Hz rounded off to the second decimal place to obtain the loss coefficient (Tδ). It is described in the examples using the format described below, with the complex viscosity obtained at a shear frequency of 10 Hz also described as a reference value. Tδ = (numerical value at a shear frequency of 1 Hz)/(numerical value at a shear frequency of 10 Hz)

### • Viscosity

Measurements were performed at 25°C by the method using a Brookfield rotational viscometer (TVB-10M, manufactured by Toki Sangyo) as described in the Japanese Industrial Standards (JIS) K 7117-1:1999.

The structures of the organopolysiloxanes used in the following examples are described using the following notations:
M is CH₃SiO_{1/2};
M^{H} is (CH₃)₂HSiO_{1/2};
M^{V} is (CH₃)₂(CH₂=CH)SiO_{1/2};
D is (CH₃)₂SiO_{2/2};
D^{H} is CH₃HSiO_{2/2};
D^{V} is CH₃(CH₂=CH)SiO_{2/2};
T is CH₃SiO_{3/2};
T^{H} is HSiO_{3/2}; and
Q is SiO_{4/2}.

### [Example 1]

In a reaction vessel, 8.5 parts by mass of the organohydrogen polysiloxane represented by the general formula (6) M^{H}₂D₅₈, 9.2 parts by mass of the alkenyl-group-containing organopolysiloxane represented by the general formula (7) M_{2.5}M^{V}_{2.5}T₃D₆₀ (weight average molecular weight: 9,520), and 8.0 × 10⁻⁵ parts by mass of chloroplatinic acid as a hydrosilylation reaction catalyst were added and mixed, and then the reaction was performed for 1 hour at 80°C. The result of the solid-or-liquid determination for the resulting crosslinked organosiloxane polymer was "a solid," with a Tδ of 0.77/1.09 and a complex viscosity of 11,100 mPa·s.

The crosslinked organosiloxane polymer obtained in Example 1 was mixed with dimethicone (INCI) that has a kinematic viscosity of 6 mm²/s at 25°C (KF-96A-6cs, manufactured by Shin-Etsu Chemical Co., Ltd.; hereinafter also referred to as 6cs) to prepare Organosiloxane Mixture 1 that has a solids concentration of 10%. At this time, the viscosity was 840 mPa·s.

### [Example 2]

In a reaction vessel, 8.4 parts by mass of the organohydrogen polysiloxane represented by the general formula (6) M^{H}₂D₅₈, 8.7 parts by mass of the alkenyl-group-containing organopolysiloxane represented by the general formula (7) M_{2.5}M^{V}_{2.5}T₃D₆₀ (weight average molecular weight: 9,520), 0.3 parts by mass of the alkenyl-group-containing organopolysiloxane represented by the general formula (8) M^{V}₂D₄₃ (weight average molecular weight: 6,950), and 8.0 × 10⁻⁵ parts by mass of chloroplatinic acid as a hydrosilylation reaction catalyst were added and mixed, and then the reaction was performed for 1 hour at 80 °C.

In the present example, two types of alkenyl-group-containing organopolysiloxanes were used in combination. The number of alkenyl groups per mole of the alkenyl-group-containing organopolysiloxanes was 2.47. The result of the solid-or-liquid determination for the resulting crosslinked organosiloxane polymer was "a solid," with a Tδ of 1.03/1.40 and a complex viscosity of 8,400 mPa·s.

The crosslinked organosiloxane polymer obtained in Example 2 was mixed with 6cs to prepare Organosiloxane Mixture 2 that has a solids content of 10%. At this time, the viscosity was 390 mPa·s.

### [Comparative Example 1]

In a reaction vessel, 8.4 parts by mass of the organohydrogen polysiloxane represented by the general formula (6) M^{H}₂D₅₈, 7.8 parts by mass of the alkenyl-group-containing organopolysiloxane represented by the general formula (7) M_{2.5}M^{V}_{2.5}T₃D₆₀ (weight average molecular weight: 9,520), 1.0 parts by mass of the alkenyl-group-containing organopolysiloxane represented by the general formula (8) M^{V}₂D₄₃, and 8.0 × 10⁻⁵ parts by mass of chloroplatinic acid as a hydrosilylation reaction catalyst were added and mixed, and then the reaction was performed for 1 hour at 80°C. In the present example, two types of alkenyl-group-containing organopolysiloxanes were used in combination. The number of alkenyl groups per mole of the alkenyl-group-containing organopolysiloxanes was 2.41. The result of the solid-or-liquid determination for the resulting crosslinked organosiloxane polymer was "a liquid," with a Tδ of 1.22/1.57 and a complex viscosity of 9,400 mPa·s.

The crosslinked organosiloxane polymer obtained in Comparative Example 1 was mixed with 6cs to prepare an organosiloxane mixture that has a solids concentration of 10%. At this time, the viscosity was 260 mPa·s.

### [Comparative Example 2]

In a reaction vessel, 6.0 parts by mass of the organohydrogen polysiloxane represented by the general formula (9) M^{H}₂D₄₀, 8.3 parts by mass of the alkenyl-group-containing organopolysiloxane represented by the general formula (7) M_{2.5}M^{V}_{2.5}T₃D₆₀ (weight average molecular weight: 9,520), 0.3 parts by mass of the alkenyl-group-containing organopolysiloxane represented by the general formula (8) M^{V}₂D₄₃ (weight average molecular weight: 6,950), and 7.0 × 10⁻⁵ parts by mass of chloroplatinic acid as a hydrosilylation reaction catalyst were added and mixed, and then the reaction was performed for 1 hour at 80°C.

In the present comparative example, two types of alkenyl-group-containing organopolysiloxanes were used in combination. The number of alkenyl groups per mole of the alkenyl-group-containing organopolysiloxanes was 2.47. The result of the solid-or-liquid determination for the resulting crosslinked organosiloxane polymer was "a solid," with a Tδ of 0.54/0.83 and a complex viscosity of 12,800 mPa·s.

The crosslinked organosiloxane polymer obtained in Comparative Example 2 was mixed with 6cs to prepare an organosiloxane mixture that has a solids concentration of 10%. At this time, the viscosity was 1,460 mPa·s.

### [Evaluation 1]

For Examples 1 and 2 and Comparative Examples 1 and 2, the W/O milky lotions were prepared using the formulations shown in Table 1 below.

**[Table 1]**

| | Component | Example | | Comparative Example | |
|---|---|---|---|---|---|
| | | 1 | 2 | 1 | 2 |
| 1 | KF-6028 (Note 1) | 0.72 | 0.72 | 0.72 | 0.72 |
| 2 | Isotridecyl Isononanoate | 6.0 | 6.0 | 6.0 | 6.0 |
| 3 | KF-96A-6cs (Note 2) | 8.28 | 8.28 | 8.28 | 8.28 |
| 4 | Mixture of cross-linked organosiloxane polymer prepared in each Example or Comparative Example with dimethicone, 10% solid concentration | 7.0 | 7.0 | 7.0 | 7.0 |
| 5 | Citric Acid | 0.2 | 0.2 | 0.2 | 0.2 |
| 6 | 1,3-BG | 10.0 | 10.0 | 10.0 | 10.0 |
| 7 | Sodium Chloride | 0.5 | 0.5 | 0.5 | 0.5 |
| 8 | Water | 67.3 | 67.3 | 67.3 | 67.3 |
| | Total | 100.0 | 100.0 | 100.0 | 100.0 |

| | | | | | |
|---|---|---|---|---|---|
| (Note 1) Shin-Etsu Chemical Co., Ltd.: PEG-9 polydimethylsiloxyethyl dimethicone (labeling name). (Note 2) Shin-Etsu Chemical Co., Ltd.: Dimethicone with a kinematic viscosity of 6 mm²/s at 25 °C (labeling name) | | | | | |

### (Preparation method)

Preparation of oil phase: Components 1 to 4 were homogeneously mixed.

Preparation of aqueous phase: Components 5 to 8 were homogeneously mixed.

The aqueous phase was gently added and transferred to the oil phase obtained above to prepare the emulsion.

### (Evaluation of characteristics)

Ten expert panelists evaluated the cosmetics with regard to the three items below, equally based on the evaluation criteria shown below. The results were determined according to the evaluation criteria described below, based on the average values provided by the ten panelists.

### (Items)

1. Initial stage of application: change at the beginning of application
2. Medium stage of application: ease of application (lubricity) during the application to the skin
3. Late stage of application: adhesion to the skin, lubricity, and a feeling of coating thickness at the end of application

### (Evaluation criteria)

5 points: very good
4 points: good
3 points: fair
2 points: slightly unsatisfactory
1 point: unsatisfactory

The average score obtained was determined according to the criteria below.

Average score determination:

| | |
|---|---|
| The average score obtained is 4.5 points or higher: | AA |
| The average score obtained is 3.5 points or higher and lower than 4.5 points: | A |
| The average score obtained is 2.5 points or higher and lower than 3.5 points: | B |
| The average score obtained is 1.5 points or higher and lower than 2.5 points: | C |
| The average score obtained is lower than 1.5 points: | D |

A determination of "A" or higher was considered acceptable.

**[Table 2]**

| No. | Evaluation Item | Example | | Comparative Example | |
|---|---|---|---|---|---|
| | | 1 | 2 | 1 | 2 |
| 1 | Initial stage of application | AA | AA | AA | C |
| 2 | Medium stage of application | AA | AA | B | A |
| 3 | Late stage of application | AA | AA | C | AA |

As shown in Table 2, the cosmetics including the polymers of Examples 1 and 2 have a better feeling when used at the time of application compared to the cosmetics including the polymers of Comparative Examples 1 and 2. Although the numbers of alkenyl groups in the alkenyl-group-containing organopolysiloxanes used in Example 2 and Comparative Examples 1 and 2 were almost the same, it was confirmed that the differences in the characteristics of the resulting polymers produce clear differences in the feeling when used.

### [Example 3]

In a reaction vessel, 18.5 parts by mass of the organohydrogen polysiloxane represented by the general formula (10) M₂D₃₈D^{H}_{2.32}, 2.1 parts by mass of 1,9-decadiene (molecular weight: 138), and 1.4 × 10⁻⁴ parts by mass of chloroplatinic acid as a hydrosilylation reaction catalyst were added and mixed, and then the reaction was performed for 1 hour at 80°C and for another 1 hour at 105°C. The result of the solid-or-liquid determination for the resulting crosslinked organosiloxane polymer was "a solid," with a Tδ of 0.64/1.08 and a complex viscosity of 3,200 mPa·s.

The crosslinked organosiloxane polymer obtained in Example 3 was mixed with Cetiol Ultimate (undecane (and) tridecane (INCI), manufactured by BASF) to obtain Organosiloxane Mixture 3 that has a solids concentration of 13.4%. At this time, the viscosity was 800 mPa·s.

### [Example 4]

In a reaction vessel, 8.9 parts by mass of the organohydrogen polysiloxane represented by the general formula (11) M₂D₃₅D^{H}_{2.24}, 1.1 parts by mass of 1,9-decadiene (molecular weight: 138), and 5.0 × 10⁻⁵ parts by mass of chloroplatinic acid as a hydrosilylation reaction catalyst were added and mixed, and then the reaction was performed for 1 hour at 80°C and for another 1 hour at 105°C. The result of the solid-or-liquid determination for the resulting crosslinked organosiloxane polymer was "a solid," with a Tδ of 0.80/1.29 and a complex viscosity of 2,300 mPa·s.

The crosslinked organosiloxane polymer obtained in Example 4 was mixed with Cetiol Ultimate to obtain Organosiloxane Mixture 4 that has a solids concentration of 13.6%. At this time, the viscosity was 360 mPa·s.

### [Comparative Example 3]

In a reaction vessel, 18.9 parts by mass of the organohydrogen polysiloxane represented by the general formula (11) M₂D₃₅D^{H}_{2.24}, 2.0 parts by mass of 1,9-decadiene (molecular weight: 138), 0.15 parts by mass of 1-hexene, and 1.2 × 10⁻⁴ parts by mass of chloroplatinic acid as a hydrosilylation reaction catalyst were added and mixed, and then the reaction was performed for 1 hour at 80°C and for another 1 hour at 105°C. The resulting crosslinked organosiloxane polymer was fluid, and the result of the solid-or-liquid determination was "a liquid," with a Tδ of 3.25/3.05 and a complex viscosity of 1,600 mPa·s.

The crosslinked organosiloxane polymer obtained in Comparative Example 3 was mixed with Cetiol Ultimate to obtain an organosiloxane mixture that has a solids concentration of 25.5%. At this time, the viscosity was 65 mPa·s.

### [Comparative Example 4]

In a reaction vessel, 10.9 parts by mass of the organohydrogen polysiloxane represented by the general formula (12) M₂D₅₁D^{H}_{2.74}, 1.1 parts by mass of 1,9-decadiene (molecular weight: 138), and 6.0 × 10⁻⁵ parts by mass of chloroplatinic acid as a hydrosilylation reaction catalyst were added and mixed, and then the reaction was performed for 1 hour at 80°C and for another 1 hour at 105°C. The result of the solid-or-liquid determination for the resulting crosslinked organosiloxane polymer was "a solid," with a loss coefficient Tδ of 0.22/0.47 and a complex viscosity of 11,300 mPa·s.

The crosslinked organosiloxane polymer obtained in Comparative Example 4 was mixed with Cetiol Ultimate to obtain an organosiloxane mixture that has a solids concentration of 13.9%. At this time, the viscosity was 6,100 mPa·s.

### [Evaluation 2]

For Examples 3 and 4 and Comparative Examples 3 and 4, the W/O milky lotions were prepared using the formulations shown in Table 3 described below.

**Table 3]**

| | Component | Example | | Comparative Example | |
|---|---|---|---|---|---|
| | | 3 | 4 | 3 | 4 |
| 1 | KF-6038 (Note 3) | 1.0 | 1.0 | 1.0 | 1.0 |
| 2 | Cetiol Ultimate | | | 2.3 | |
| 3 | Isopropyl Myristate | 6.0 | 6.0 | 6.0 | 6.0 |
| 4 | SMARTS (Note 4) | 9.8 | 9.9 | 10.0 | 10.0 |
| 5 | Mixtures of cross-linked organosiloxane polymers prepared in each Example and each Comparative Example with undecane and tridecane, solid concentration is as above | 5.2 | 5.1 | 2.7 | 5.0 |
| 6 | Citric Acid | 0.2 | 0.2 | 0.2 | 0.2 |
| 7 | 1,3-BG | 10.0 | 10.0 | 10.0 | 10.0 |
| 8 | Sodium Chloride | 0.5 | 0.5 | 0.5 | 0.5 |
| 9 | Water | 67.3 | 67.3 | 67.3 | 67.3 |
| | Total | 100.0 | 100.0 | 100.0 | 100.0 |

| | | | | | |
|---|---|---|---|---|---|
| (Note 3) Shin-Etsu Chemical Co., Ltd.: lauryl PEG-9 polydimethylsiloxyethyl dimethicone (labeling name). (Note 4) Nikko Chemicals: Isododecane, Hydrogenated tetradecenyl/methylpentadecane (labeling name). | | | | | |

### (Preparation method)

Preparation of oil phase: Components 1 to 5 were homogeneously mixed.

Preparation of aqueous phase: Components 6 to 9 were homogeneously mixed.

The aqueous phase was gently added and transferred to the oil phase obtained above to prepare the emulsion.

### (Evaluation of characteristics)

Ten expert panelists evaluated the cosmetics with regard to the three items below, equally based on the evaluation criteria shown below. The results were determined according to the evaluation criteria described below, based on the average values provided by the ten panelists. The results are shown in Table 4.

### (Items)

1. Beginning of application: change at the beginning of application
2. Time of application: ease of application (lubricity) during the application to the skin
3. Late stage of application: adhesion to the skin, lubricity, and a feeling of coating thickness at the end of application

### (Evaluation criteria)

5 points: very good
4 points: good
3 points: fair
2 points: slightly unsatisfactory
1 point: unsatisfactory

The average score obtained was determined according to the criteria described below.

### Average score determination:

| | |
|---|---|
| The average score obtained is 4.5 points or higher: | AA |
| The average score obtained is 3.5 points or higher and lower than 4.5 points: | A |
| The average score obtained is 2.5 points or higher and lower than 3.5 points: | B |
| The average score obtained is 1.5 points or higher and lower than 2.5 points: | C |
| The average score obtained is lower than 1.5 points: | D |

A determination of "A" or higher was considered acceptable.

**[Table 4]**

| No. | Evaluation Item | Example | | Comparative Example | |
|---|---|---|---|---|---|
| | | 3 | 4 | 3 | 4 |
| 1 | Initial stage of application | AA | AA | A | C |
| 2 | Medium stage of application | AA | AA | B | B |
| 3 | Late stage of application | AA | AA | C | A |

As shown in Table 4, the cosmetics including the polymers obtained in Examples 3 and 4 have a better feeling when used at the time of application compared to the cosmetics including the polymers of Comparative Examples 1 and 2.

Comparative Example 3, which had a Tδ of 3.3/3.1 and a result of the solid-or-liquid determination of "a liquid," showed a little oil on the surface one week after the date of preparation, indicating low stability. Although the structures of the ingredients and the numbers of hydrosilyl groups in the ingredients were almost the same in Examples 3 and 4 and Comparative Examples 3 and 4, it was confirmed that the differences in the characteristics of the resulting polymers affect the texture.

### [Example 5]

In a reaction vessel, 109.6 parts by mass of the organohydrogen polysiloxane represented by the general formula (13) M₂D₂₉D^{H}_{1.4}, 20.6 parts by mass of diallyl polyether represented by the general formula (14) CH₂=CH-CH₂-(OC₂H₄)₁₂-O-CH₂-CH=CH₂ (weight average molecular weight: 770), 32.6 parts by mass of isopropyl alcohol, and 6.0 × 10⁻⁴ parts by mass of chloroplatinic acid as a hydrosilylation reaction catalyst were added and mixed, and then the reaction was performed for 3 hours and 30 minutes at 80°C. Next, 26 g of a 2% aqueous citric acid solution was added and heated for 1 hour, 10.4 g of a 5% aqueous sodium bicarbonate solution was added and heated for 1 hour, and then 0.09 g of d-δ-tocopherol was added as an antioxidant. The isopropyl alcohol was removed under reduced pressure to obtain a crosslinked organosiloxane polymer. The result of the solid-or-liquid determination for the resulting crosslinked organosiloxane polymer was "a solid," with a Tδ of 0.77/0.97 and a complex viscosity of 13,600 mPa·s.

The crosslinked organosiloxane polymer obtained in Example 5 was mixed with dimethicone (INCI) that has a kinematic viscosity of 6 mm²/s at 25°C (KF-96A-6cs, manufactured by Shin-Etsu Chemical Co., Ltd.) to obtain Organosiloxane Mixture 5 that has a solids concentration of 24%. At this time, the viscosity was 1,700 mPa·s.

### [Example 6]

In a reaction vessel, 90.5 parts by mass of the organohydrogen polysiloxane represented by the general formula (15) M₂D₂₆D^{H}_{1.08}, 8.7 parts by mass of allyl polyglycerin represented by the general formula (16) below (weight average molecular weight: 300), 25.0 parts by mass of isopropyl alcohol, and 2.0 × 10⁻⁴ parts by mass of chloroplatinic acid as a hydrosilylation reaction catalyst were added and mixed, and then the reaction was performed for 4 hours at 80°C. Next, 19.8 g of a 2% aqueous citric acid solution was added and heated for 60 minutes, 9.9 parts by mass of a 5% aqueous sodium bicarbonate solution was added and heated for 60 minutes, and then 0.1 g of d-δ-tocopherol was added as an antioxidant. The isopropyl alcohol was removed under reduced pressure to obtain a crosslinked organosiloxane polymer.

The result of the solid-or-liquid determination for the resulting crosslinked organosiloxane polymer was "a solid," with a Tδ of 0.74/1.14 and a complex viscosity of 12,600 mPa·s.

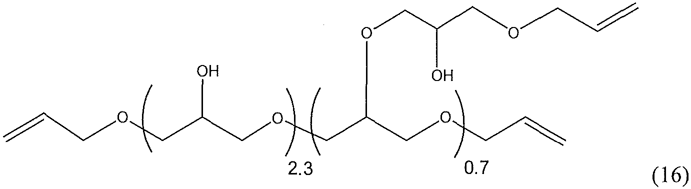

The crosslinked organosiloxane polymer obtained in Example 6 was mixed with KF-96A-6cs to obtain Organosiloxane Mixture 6 that has a solids concentration of 24%. At this time, the viscosity was 1,000 mPa·s.

### [Comparative Example 5]

In a reaction vessel, 101.8 parts by mass of the organohydrogen polysiloxane represented by the general formula (17) M₂D₃₀D^{H}_{1.6}, 20.6 parts by mass of diallyl polyether represented by the general formula (14) CH₂=CH-CH₂-(OC₂H₄)₁₂-O-CH₂-CH=CH₂ (weight average molecular weight: 770), 30.6 parts by mass of isopropyl alcohol, and 6.0 × 10⁻⁴ parts by mass of chloroplatinic acid as a hydrosilylation reaction catalyst were added and mixed, and then the reaction was performed for 4 hours and 30 minutes at 80°C. Next, 24.5 g of a 2% aqueous citric acid solution was added and heated for 1 hour, 9.8 g of a 5% aqueous sodium bicarbonate solution was added and heated for 1 hour, and then 0.02 g of d-δ-tocopherol was added as an antioxidant. The isopropyl alcohol was removed under reduced pressure to obtain a crosslinked organosiloxane polymer. The result of the solid-or-liquid determination for the resulting crosslinked organosiloxane polymer was "a solid," with a Tδ of 0.64/0.87 and a complex viscosity of 15,800 mPa·s.

The crosslinked organosiloxane polymer obtained in Comparative Example 5 was mixed with KF-96A-6cs to obtain an organosiloxane mixture that has a solids concentration of 24%. At this time, the viscosity was 1,390 mPa·s.

### [Comparative Example 6]

In a reaction vessel, 99.9 parts by mass of the organohydrogen polysiloxane represented by the general formula (15) M₂D₂₅D^{H}₁, 8.7 parts by mass of allyl polyglycerin represented by the general formula (16) (weight average molecular weight: 300), 25.4 parts by mass of isopropyl alcohol, and 2.0 × 10⁻⁴ parts by mass of chloroplatinic acid as a hydrosilylation reaction catalyst were added and mixed, and then the reaction was performed for 4 hours at 80°C. Next, 21.7 parts by mass of a 2% aqueous citric acid solution was added and heated for 1 hour, 10.9 parts by mass of a 5% aqueous sodium bicarbonate solution was added and heated for 1 hour, and then 0.09 g of d-δ-tocopherol was added as an antioxidant. The isopropyl alcohol was removed under reduced pressure to obtain a crosslinked organosiloxane polymer. The result of the solid-or-liquid determination for the resulting crosslinked organosiloxane polymer was "a liquid," with a Tδ of 1.19/1.54 and a complex viscosity of 8,400 mPa·s.

The crosslinked organosiloxane polymer obtained in Comparative Example 6 was mixed with KF-96A-6cs to obtain an organosiloxane mixture that has a solids concentration of 24%. At this time, the viscosity was 610 mPa·s.

### [Evaluation 3]

For Examples 5 and 6 and Comparative Examples 5 and 6, the W/O milky lotions were prepared using the formulations shown in Table 5 described below.

**[Table 5]**

| | Component | Example | | Comparative Example | | | |
|---|---|---|---|---|---|---|---|
| | | 5 | 6 | 5 | 6 | 7 | 8 |
| 1 | KF-6028 (Note 1) | | | | | 0.8 | 0.2 |
| 2 | Isotridecyl Isononanoate | 6.0 | 6.0 | 6.0 | 6.0 | 6.0 | |
| 3 | KF-96A-6cs (Note 2) | 13.0 | 13.0 | 13.0 | 13.0 | 15.2 | 13.0 |
| 4 | Organosiloxane mixture with a concentration of 24% prepared in the examples and comparative examples | 3.0 | 3.0 | 3.0 | 3.0 | - | 2.8* |
| 5 | Citric Acid | 0.2 | 0.2 | 0.2 | 0.2 | 0.2 | 0.2 |
| 6 | 1,3-BG | 10.0 | 10.0 | 10.0 | 10.0 | 10.0 | 10.0 |
| 7 | Sodium Chloride | 0.5 | 0.5 | 0.5 | 0.5 | 0.5 | 0.5 |
| 8 | Water | 67.3 | 67.3 | 67.3 | 67.3 | 67.3 | 67.3 |
| | Total | 100.0 | 100.0 | 100.0 | 100.0 | 100.0 | 100.0 |

| | | | | | | | |
|---|---|---|---|---|---|---|---|
| * Organosiloxane mixture with 24% solids concentration of cross-linked organosiloxane polymer in Comparative Example 5. (Note 1) Shin-Etsu Chemical Co., Ltd.: PEG-9 polydimethylsiloxyethyl dimethicone (labeling name) (Note 2) Shin-Etsu Chemical Co., Ltd.: Dimethicone with a kinematic viscosity of 6 mm²/s at 25 °C (labeling name) | | | | | | | |

### (Preparation method)

Preparation of oil phase: Components 1 to 4 were homogeneously mixed.

Preparation of aqueous phase: Components 5 to 8 were homogeneously mixed.

The aqueous phase was gently added and transferred to the oil phase to prepare the emulsion.

### (Evaluation of characteristics)

Ten expert panelists evaluated the cosmetics with regard to the three items below, equally based on the evaluation criteria shown below. The results were determined according to the evaluation criteria described below, based on the average values provided by the ten panelists. The results are shown in Table 6.

### (Items)

1. Initial stage of application: change at the beginning of application
2. Medium stage of application: ease of application (lubricity) during the application to the skin
3. Late stage of application: adhesion to the skin, lubricity, and a feeling of coating thickness at the end of application

### (Evaluation criteria)

5 points: very good
4 points: good
3 points: fair
2 points: slightly poor
1 point: poor

The average score obtained was determined according to the criteria below.

### Average score determination:

| | |
|---|---|
| The average score obtained is 4.5 points or higher: | AA |
| The average score obtained is 3.5 points or higher and lower than 4.5 points: | A |
| The average score obtained is 2.5 points or higher and lower than 3.5 points: | B |
| The average score obtained is 1.5 points or higher and lower than 2.5 points: | C |
| The average score obtained is lower than 1.5 points: | D |

A determination of "A" or higher was considered acceptable.

**[Table 6]**

| No. | Evaluation Item | Example | | Comparative Example | | | |
|---|---|---|---|---|---|---|---|
| | | 5 | 6 | 5 | 6 | 7 | 8 |
| (1) | Initial stage of application | AA | AA | C | A | B | A |
| (2) | Medium stage of application | AA | AA | A | B | B | B |
| (3) | Late stage of application | AA | AA | AA | C | C | B |

As shown in Table 6, the cosmetics including the polymers of Examples 5 and 6 have a better feeling when used at the time of application compared to the cosmetics including the polymers of Comparative Examples 5 and 6. The cosmetic including the polymer of Comparative Example 7, which does not use a crosslinked resin, has an inferior texture at the beginning of application and after application. Mixing KF-6028, which does not have a crosslinked structure, with the cosmetic including the polymer of Comparative Example 8, which has a low Tδ value, does not obtain evaluation results as good as those in the examples.

### [Example 7]

In a reaction vessel, 70.0 parts by mass of the organohydrogen polysiloxane represented by the general formula (17) below, 23.4 parts by mass of the alkenyl-group-containing organopolysiloxane represented by the general formula (8) M^{V}₂D₄₃ (weight average molecular weight: 6,950), and 1.6 × 10⁻⁴ parts by mass of chloroplatinic acid as a hydrosilylation reaction catalyst were added and mixed, and then the reaction was performed for 2 hours at 100°C. The result of the solid-or-liquid determination for the resulting crosslinked organosiloxane polymer was "a solid," with a Tδ of 0.69/0.91 and a complex viscosity of 11,400 mPa·s.

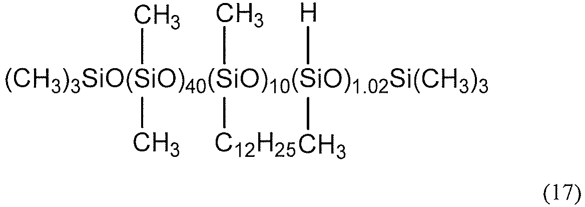

### [Comparative Example 7]

In a reaction vessel, 20.0 parts by mass of the organohydrogen polysiloxane represented by the general formula (18) below, 9.2 parts by mass of the alkenyl-group-containing organopolysiloxane represented by the general formula (8) M^{V}₂D₄₃ (weight average molecular weight: 6,950), and 6.0 × 10⁻⁵ parts by mass of chloroplatinic acid as a hydrosilylation reaction catalyst were added and mixed, and then the reaction was performed for 2 hours at 100°C. The result of the solid-or-liquid determination for the resulting crosslinked organosiloxane polymer was "a solid," with a Tδ of 0.14/0.40 and a complex viscosity of 31,600 mPa·s.

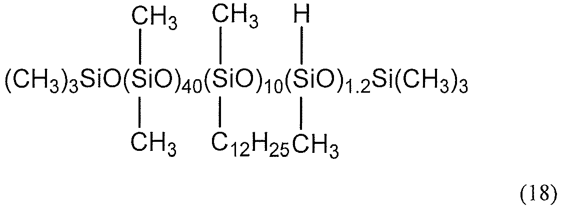

### [Evaluation 4]

For Example 7 and Comparative Example 7, the O/W liquid foundations were prepared using the formulations shown in Table 7 described below.

**[Table 7]**

| No | Component | Example | Comparative Example |
|---|---|---|---|
| | | 7 | 7 |
| 1 | Cetyl PEG/PPG-10/1 dimethicone, HLB10 | 2 | 2 |
| 2 | 1,3-BG | 8.0 | 8.0 |
| 3 | Squalane | 11.7 | 11.7 |
| 4 | Organosiloxane prepared in Example or Comparative Example | 2.0 | 2.0 |
| 5 | KF-96A-6cs (Note 1) | 6.0 | 6.0 |
| 6 | Isostearic Acid | 0.3 | 0.3 |
| 7 | 1% aqueous sodium hydroxide solution | 4.2 | 4.2 |
| 8 | Water | 25.8 | 25.8 |
| 9 | Aristoflex (5%) | 20.0 | 20.0 |
| 10 | Pigment dispersion (Note 2) | 11.6 | 11.6 |
| 11 | Water | 8.4 | 8.4 |
| | Total | 100.0 | 100.0 |

| | | | |
|---|---|---|---|
| (Note 1) Shin-Etsu Chemical Co., Ltd.: Dimethicone with a kinematic viscosity of 6 mm²/s at 25 °C (labeling name) (Note 2) Pigment dispersion prepared by mixing the following components: | | | |

| | |
|---|---|
| • Titanium dioxide treated with KF-9901, ex Shin-Etsu Chemical Co., Ltd.: | 7.0 g |
| • Iron oxide (red) treated with KF-9901, ex Shin-Etsu Chemical Co., Ltd. : | 0.3 g |
| • Iron oxide (yellow) treated with KF-9901, ex Shin-Etsu Chemical Co., Ltd. : | 1.0 g |
| • Iron oxide (black) treated with KF-9901, ex Shin-Etsu Chemical Co., Ltd. : | 0.1 g |
| • PEG-9 dimethicone (HLB: 10) : | 0.3 g |
| • 1,3- BG : | 2.9 g |

### (Preparation method)

A: Each of the pigment dispersions of Components 4, 5, and 10 were homogeneously mixed.
B: Components 1 and 2 were mixed.
C: Components 3 to 6 were mixed.
D: Components 7 and 8 were mixed.
E. The mixture of C was added to the mixture of B, the resultant was emulsified, and the mixture of D was added.
F. Components 9 to 11 were added to the mixture of E to obtain the O/W liquid foundation.

### (Evaluation of characteristics)

Ten expert panelists evaluated the cosmetics with regard to the three items below, equally based on the evaluation criteria shown below. The results were determined according to the evaluation criteria described below, based on the average values provided by the ten panelists. The results are shown in Table 6.

### (Items)

1. Initial stage of application: change at the beginning of application
2. Medium stage of application: ease of application (lubricity) during the application to the skin
3. Late stage of application: adhesion to the skin, lubricity, and a feeling of coating thickness at the end of application

### (Evaluation criteria)

5 points: very good
4 points: good
3 points: fair
2 points: slightly poor
1 point: poor

The average score obtained was determined according to the criteria described below.

### Average score determination:

| | |
|---|---|
| The average score obtained is 4.5 points or higher: | AA |
| The average score obtained is 3.5 points or higher and lower than 4.5 points: | A |
| The average score obtained is 2.5 points or higher and lower than 3.5 points: | B |
| The average score obtained is 1.5 points or higher and lower than 2.5 points: | C |
| The average score obtained is lower than 1.5 points: | D |

A determination of "A" or higher was considered acceptable.

**[Table 8]**

| No. | Evaluation item | Example | Comparative Example |
|---|---|---|---|
| | | 7 | 7 |
| 1 | Initial stage of application | AA | A |
| 2 | Medium stage of application | AA | A |
| 3 | Late stage of application | AA | B |

As shown in Table 8, the cosmetic including the polymer of Example 7 has better adhesion to the skin, better lubricity, and a better feeling of coating thickness at the end of application compared to the cosmetic including the polymer of Comparative Example 7.

As described above, the results of Evaluations 1 to 4 confirm that the crosslinked organosiloxane polymer that has a crosslinked structure including a carbon-silicon bond, a loss coefficient of 1.1 or less at a measurement temperature of 25°C, a shear strain of 10%, and a shear frequency of 1 Hz, and a loss coefficient of 0.9 to 1.4 at a measurement temperature of 25°C, a shear strain of 10%, and a shear frequency of 10 Hz can provide cosmetics with a good texture, regardless of the structure and types of functional groups.

### [Formulation Example 1]

### W/O sunscreen cream

### <Preparation of the cosmetic>

A: Components 9 to 11 were homogeneously dispersed using a disper, Components 1 to 8 were added, and the resultant was homogeneously mixed.
B: Components 12 to 17 were homogeneously mixed.
C: The mixture B was added to the mixture A, and the resultant was emulsified to obtain the W/O sunscreen cream.

| Components | Mass (%) |
|---|---|
| 1. Organosiloxane Mixture 5 | 3 |
| 2. Organosiloxane Mixture 3 | 2 |
| 3. KF-6048 (Note 1) | 2.5 |
| 4. Disteardimonium hectorite | 0.3 |
| 5. Ethylhexyl methoxycinnamate | 7.5 |
| 6. Octocrylene | 3 |
| 7. Homosalate | 5 |
| 8. Dicaprylyl carbonate | 7 |
| 9. TMF-1.5 (Note 2) | 10.5 |
| 10. KF-6105 (Note 3) | 1.5 |
| 11. Zinc oxide fine particles treated with alkylsilane (Note 4) | 18 |
| 12. Butylene glycol | 3 |
| 13. Ethanol | 5 |
| 14. Sodium citrate | 0.2 |
| 15. Sodium chloride | 1 |
| 16. Allantoin | 0.2 |
| 17. Purified water | Remaining amount |
| Total | 100 |

| | |
|---|---|
| (Note 1) Cetyl PEG/PPG-10/1 dimethicone (labeling name), manufactured by Shin-Etsu Chemical Co., Ltd. (Note 2) Methyl trimethicone (labeling name), manufactured by Shin-Etsu Chemical Co., Ltd. (Note 3) Lauryl polyglyceryl-3 polydimethylsiloxyethyl dimethicone (labeling name), manufactured by Shin-Etsu Chemical Co., Ltd. (Note 4) Treated with AES-3083, manufactured by Shin-Etsu Chemical Co., Ltd. | |

The resulting W/O sunscreen cream had a good feeling when used, good applicability, and excellent storage stability.

### [Formulation Example 2]

### W/O shaking sunscreen

### <Preparation of the cosmetic>

A: Components 5 to 10 and 11 to 14 were heated and dissolved, then homogeneously mixed with Components 1 to 4.
B: Components 15 to 19 were homogeneously mixed.
C: B was added to A, and the resultant was emulsified to obtain the W/O shaking sunscreen.

| Components | Mass (%) |
|---|---|
| 1. Organosiloxane Mixture 4 | 2 |
| 2. KF-6048 (Note 1) | 2.5 |
| 3. Disteardimonium hectorite | 0.2 |
| 4. KMP-590 (Note 2) | 2 |
| 5. Ethylhexyl methoxycinnamate | 7.5 |
| 6. Octocrylene | 5 |
| 7. Homosalate | 5 |
| 8. Diethylamino hydroxybenzoyl hexyl benzoate | 2.5 |
| 9. Bis-ethylhexyloxyphenol methoxyphenyl triazine | 2 |
| 10. Ethylhexyl salicylate | 5 |
| 11. Coco-caprylate/caprate | 12 |
| 12. Ethyl isostearate | 10 |
| 13. Hexyl laurate | 8 |
| 14. Stearyl glycyrrhizate | 0.2 |
| 15. Butylene glycol | 3 |
| 16. Ethanol | 6 |
| 17. Sodium citrate | 0.2 |
| 18. Sodium chloride | 1 |
| 19. Purified water | Remaining amount |
| Total | 100 |

| | |
|---|---|
| (Note 1) Cetyl PEG/PPG-10/1 dimethicone (labeling name), manufactured by Shin-Etsu Chemical Co., Ltd. (Note 2) Polymethylsilsesquioxane (labeling name), manufactured by Shin-Etsu Chemical Co., Ltd. | |

The resulting W/O shaking sunscreen had a good feeling when used, good applicability, and excellent storage stability.

### [Formulation Example 3]

### W/O eye cream

### <Preparation of the cosmetic>

A: Components 1 to 9 were homogeneously mixed.
B: Components 10 to 16 were homogeneously mixed.
C: B was added to A, and the resultant was emulsified to obtain the eye cream.

| Components | Mass (%) |
|---|---|
| 1. KSG-840 (Note 1) | 1.8 |
| 2. Organosiloxane Mixture 6 | 1.2 |
| 3. Organosiloxane Mixture 1 | 3 |
| 4. Organosiloxane Mixture 4 | 3 |
| 5. KF-6105 (Note 2) | 1.5 |
| 6. Simmondsia chinensis (jojoba) seed oil | 6 |
| 7. Limnanthes alba (meadowfoam) seed oil | 12 |
| 8. Butyrospermum parkii (shea) butter | 1 |
| 9. KSP-300 (Note 3) | 2 |
| 10. Sodium hyaluronate | 0.1 |
| 11. Erythritol | 5 |
| 12. Methyl gluceth-10 | 3 |
| 13. Phenoxyethanol | 0.3 |
| 14. Pentylene glycol | 3 |
| 15. Sodium chloride | 0.5 |
| 16. Water | Remaining amount |
| Total | 100 |

| | |
|---|---|
| (Note 1) Mixture of 30% lauryl dimethicone/polyglycerin-3 crosspolymer (labeling name) and 70% squalane (labeling name), manufactured by Shin-Etsu Chemical Co., Ltd. (Note 2) Lauryl polyglyceryl-3 polydimethylsiloxyethyl dimethicone (labeling name), manufactured by Shin-Etsu Chemical Co., Ltd. (Note 3) Diphenyl dimethicone/vinyl diphenyl dimethicone/silsesquioxane crosspolymer (labeling name), manufactured by Shin-Etsu Chemical Co., Ltd. | |

The resulting W/O eye cream had a good feeling when used, good applicability, and excellent storage stability.

### [Formulation Example 4]

### O/W milky lotion

### <Preparation of the cosmetic>

A: Components 1 to 6 were homogeneously mixed at 90°C.
B: Components 7 to 12 were homogeneously mixed at 85°C.
C: B was added to A, the resultant was emulsified and cooled, then Component 13 was added, and the resultant was homogeneously mixed to obtain the milky lotion.

| Components | Mass (%) |
|---|---|
| 1. Butylene glycol | 6 |
| 2. PEG-20 glyceryl isostearate | 1.2 |
| 3. Ethylhexyl palmitate | 4 |
| 4. Organosiloxane Mixture 2 | 2 |
| 5. Polyquaternium-51 | 0.1 |
| 6. Tocopherol | 0.05 |
| 7. Diglycerin | 2 |
| 8. Ethylhexylglycerin | 0.1 |
| 9. Glycerin | 8 |
| 10. Sorbitol | 3 |
| 11. Alkyl-modified carbomer | 0.2 |
| 12. Water | Remaining amount |
| 13. 10% aqueous potassium hydroxide solution | 0.5 |
| Total | 100 |

The resulting O/W milky lotion had a good feeling when used, good applicability, and excellent storage stability.

### [Formulation Example 5]

### Balm

### <Preparation of the cosmetic>

A: Components 1 to 11 were homogeneously mixed at 90°C, and the resultant was cooled to obtain the balm.

| Components | Mass (%) |
|---|---|
| 1. Petrolatum | 27 |
| 2. Squalane | Remaining amount |
| 3. KSP-101 (Note 1) | 10 |
| 4. KMP-590 (Note 2) | 6 |
| 5. Organosiloxane Mixture 4 | 5 |
| 6. Organosiloxane Mixture 2 | 3 |
| 7. Dimethicone (6cs) | 7 |
| 8. KF-56A (Note 3) | 4 |
| 9. KP-561P (Note 4) | 2 |
| 10. KF-6038 (Note 5) | 0.2 |
| 11. Dextrin palmitate | 10 |
| Total | 100 |

| | |
|---|---|
| (Note 1) Vinyl dimethicone/methicone silsesquioxane crosspolymer (labeling name), manufactured by Shin-Etsu Chemical Co., Ltd. (Note 2) Polymethylsilsesquioxane (labeling name), manufactured by Shin-Etsu Chemical Co., Ltd. (Note 3) Diphenylsiloxy phenyl dimethicone (labeling name), manufactured by Shin-Etsu Chemical Co., Ltd. (Note 4) Acrylates/stearyl acrylate/dimethicone methacrylate copolymer (labeling name), manufactured by Shin-Etsu Chemical Co., Ltd. (Note 5) Lauryl PEG-9 polydimethylsiloxyethyl dimethicone (labeling name), manufactured by Shin-Etsu Chemical Co., Ltd. | |

The resulting balm had a good feeling when used, good applicability, and excellent storage stability.

### [Formulation Example 6]

### W/O sunscreen milk

### <Preparation of the cosmetic>

A: Components 1 to 7 were homogeneously mixed.
B: Components 10 to 16 were homogeneously mixed.
C: B was added to A, the resultant was emulsified, Components 8 and 9 were added, and the resultant was homogeneously mixed to obtain the W/O sunscreen milk.

| Components | Mass (%) |
|---|---|
| 1. Organosiloxane Mixture 5 | 3 |
| 2. Organosiloxane Mixture 2 | 2 |
| 3. KF-6028 (Note 2) | 1 |
| 4. Simmondsia chinensis (jojoba) seed oil | 2 |
| 5. Dimethicone (6cs) | 5 |
| 6. KF-9021L (Note 3) | 2 |
| 7. Isotridecyl isononanoate | 5 |
| 8. SPD-TSL (Note 4) | 20 |
| 9. SPD-Z5L (Note 5) | 28 |
| 10. Dipropylene glycol | 2 |
| 11. Sodium citrate | 0.2 |
| 12. Sodium chloride | 1 |
| 13. Arbutin | 2 |
| 14. Dipotassium glycyrrhizate | 0.2 |
| 15. Sodium metabisulfite | 0.05 |
| 16. Purified water | Remaining amount |
| Total | 100 |

| | |
|---|---|
| (Note 1) Mixture of 25% dimethicone/PEG-10/15 crosspolymer (labeling name) and 75% dimethicone, manufactured by Shin-Etsu Chemical Co., Ltd. (Note 2) PEG-9 polydimethylsiloxyethyl dimethicone (labeling name), manufactured by Shin-Etsu Chemical Co., Ltd. (Note 3) Trimethylsiloxysilicate (labeling name), manufactured by Shin-Etsu Chemical Co., Ltd. (Note 4) 40% titanium dioxide dispersion, manufactured by Shin-Etsu Chemical Co., Ltd. (Note 5) 60% zinc oxide dispersion, manufactured by Shin-Etsu Chemical Co., Ltd. | |

The resulting W/O sunscreen milk had a good feeling when used, good applicability, and excellent storage stability.

### [Formulation Example 7]

### O/W sunscreen cream

### <Preparation of the cosmetic>

A: Components 1 to 6 were heated to 85°C and homogeneously mixed.
B: Components 7 to 15 were heated to 85°C and homogeneously mixed.
C: The mixture B was added to the mixture A, and the resultant was emulsified at 85°C and then slowly cooled while stirred to obtain the sunscreen cream.

| Components | Mass (%) |
|---|---|
| 1. Hydroxyethylcellulose | 0.1 |
| 2. Ethanol | 10 |
| 3. Butylene glycol | 6 |
| 4. Allantoin | 0.1 |
| 5. SIMULGEL EG (Note 1) | 2.5 |
| 6. Water | Remaining amount |
| 7. KF-56A (Note 2) | 3 |
| 8. Organosiloxane Mixture 4 | 1 |
| 9. Cetyl alcohol | 2 |
| 10. Ethylhexyl methoxycinnamate | 7.5 |
| 11. Diethylamino hydroxybenzoyl hexyl benzoate | 2 |
| 12. Ethylhexyl salicylate | 5 |
| 13. Polyoxyethylene (60) hydrogenated castor oil | 1 |
| 14. KF-6011 (Note 3) | 0.5 |
| 15. Tocopherol | 0.05 |
| Total | 100 |

| | |
|---|---|
| (Note 1) Sodium acrylate/sodium acryloyldimethyl taurate copolymer composition, manufactured by Seppic (Note 2) Diphenylsiloxy phenyl trimethicone (labeling name), manufactured by Shin-Etsu Chemical Co., Ltd. (Note 3) PEG-11 methyl ether dimethicone (labeling name), manufactured by Shin-Etsu Chemical Co., Ltd. | |

The resulting O/W sunscreen cream had a good feeling when used, good applicability, and excellent storage stability.

### [Formulation Example 8]

### Sunscreen gel

### <Preparation of the cosmetic>

A: Components 1 to 10 were homogeneously mixed to obtain the sunscreen gel.

| Components | Mass (%) |
|---|---|
| 1. KSG-42A (Note 1) | 10 |
| 2. Crosslinked organosiloxane polymer of Example 4 | 5 |
| 3. KSG-042Z (Note 2) | Remaining amount |
| 4. KF-56A (Note 3) | 27.5 |
| 5. Octocrylene | 2 |
| 6. Homosalate | 10 |
| 7. Ethylhexyl salicylate | 5 |
| 8. Bis-ethylhexyloxyphenol methoxyphenyl triazine | 3 |
| 9. KSP-441 (Note 4) | 10 |
| 10. KMP-592 (Note 5) | 2 |
| Total | 100 |

| | |
|---|---|
| (Note 1) Mixture of 20% vinyl dimethicone/lauryl dimethicone crosspolymer (labeling name) and 80% isododecane (labeling name), manufactured by Shin-Etsu Chemical Co., Ltd. (Note 2) Mixture of 20% lauryl polydimethylsiloxyethyl dimethicone/bis-vinyldimethicone crosspolymer (labeling name) and 80% isododecane (labeling name), manufactured by Shin-Etsu Chemical Co., Ltd. (Note 3) Diphenylsiloxy phenyl trimethicone (labeling name), manufactured by Shin-Etsu Chemical Co., Ltd. (Note 4) Polysilicone-22 (labeling name), manufactured by Shin-Etsu Chemical Co., Ltd. (Note 5) Methyl/phenyl polysilsesquioxane (labeling name), manufactured by Shin-Etsu Chemical Co., Ltd. | |

The resulting sunscreen gel had a good feeling when used, good applicability, excellent storage stability, and excellent transparency.

### [Formulation Example 9]

### Non-aqueous sunscreen

### <Preparation of the cosmetic>

A: Components 1 to 6 were homogeneously mixed and dissolved.
B: Components 7 to 11 were homogeneously mixed.
C: The mixtures of A and B were homogeneously mixed to obtain the non-aqueous sunscreen.

| Components | Mass (%) |
|---|---|
| 1. Dimethicone (2cs) | Remaining amount |
| 2. Octocrylene | 5 |
| 3. Diethylamino hydroxybenzoyl hexyl benzoate | 2 |
| 4. Ethylhexyl salicylate | 5 |
| 5. Stearyl glycyrrhetinate | 0.2 |
| 6. BHT | 0.1 |
| 7. Crosslinked organosiloxane polymer of Example 5 | 2 |
| 8. Crosslinked organosiloxane polymer of Example 4 | 3 |
| 9. KF-56A (Note 1) | 12 |
| 10. SPD-Z5L (Note 2) | 58 |
| 11. KSP-105 (Note 3) | 4 |
| Total | 100 |

| | |
|---|---|
| (Note 1) Diphenylsiloxy phenyl trimethicone (labeling name), manufactured by Shin-Etsu Chemical Co., Ltd. (Note 2) 60% zinc oxide dispersion, manufactured by Shin-Etsu Chemical Co., Ltd. (Note 3) Vinyl dimethicone/methicone silsesquioxane crosspolymer (labeling name), manufactured by Shin-Etsu Chemical Co., Ltd. | |

The resulting non-aqueous sunscreen had a good feeling when used, good applicability, and excellent storage stability.

### [Formulation Example 10]

### W/O liquid foundation

### <Preparation of the cosmetic>

A: Components 9 to 14 were dispersed using a three-roll mill.
B: Components 1 to 8 were homogeneously mixed.
C: Components 15 to 18 were homogeneously mixed.
D: C was added to B, the resultant was emulsified, and then A was added to obtain the W/O liquid foundation.

| Components | Mass (%) |
|---|---|
| 1. Organosiloxane Mixture 5 | 3.5 |
| 2. KSG-016F (Note 1) | 5 |
| 3. KF-6038 (Note 2) | 2.5 |
| 4. Organically modified clay mineral | 1 |
| 5. Dimethicone (2cs) | 21 |
| 6. Octocrylene | 5 |
| 7. Polysilicone-15 | 2 |
| 8. KSP-101 (Note 3) | 2 |
| 9. Isononyl isononanoate | 3.7 |
| 10. KP-578 (Note 4) | 0.3 |
| 11. KTP-09W (Note 5) | 14 |
| 12. KTP-09R (Note 5) | 0.2 |
| 13. KTP-09Y (Note 5) | 0.5 |
| 14. KTP-09B (Note 5) | 1 |
| 15. Butylene glycol | 5 |
| 16. Sodium citrate | 0.2 |
| 17. Sodium chloride | 1 |
| 18. Water | Remaining amount |
| Total | 100 |

| | |
|---|---|
| (Note 1) Mixture of 25% dimethicone/vinyl dimethicone crosspolymer (labeling name) and 75% dimethicone, manufactured by Shin-Etsu Chemical Co., Ltd. (Note 2) Lauryl PEG-9 polydimethylsiloxyethyl dimethicone (labeling name), manufactured by Shin-Etsu Chemical Co., Ltd. (Note 3) Vinyl dimethicone/methicone silsesquioxane crosspolymer (labeling name), manufactured by Shin-Etsu Chemical Co., Ltd. (Note 4) Acrylates/ethylhexyl acrylate/dimethicone methacrylate copolymer (labeling name), manufactured by Shin-Etsu Chemical Co., Ltd. (Note 5) Inorganic color pigments treated with KF-9909; W (white), R (red), Y (yellow), and B (black), manufactured by Shin-Etsu Chemical Co., Ltd. | |

The resulting W/O liquid foundation had a good feeling when used, good applicability, and excellent storage stability.

### [Formulation Example 11]

### W/O solid foundation

### <Preparation of the cosmetic>

A: Components 7 to 12 were dispersed using a three-roll mill.
B: Components 1 to 6 were dissolved at 90°C, then A was added, and the resultant was homogeneously mixed.
C: Components 13 to 17 were homogeneously mixed.
D: C was added to B, and the resultant was emulsified to obtain the W/O solid foundation.

| Components | Mass (%) |
|---|---|
| 1. Organosiloxane Mixture 6 | 5 |
| 2. Organosiloxane Mixture 3 | 3 |
| 3. KF-6038 (Note 1) | 2 |
| 4. SPD-T7 (Note 2) | 10 |
| 5. KF-56A (Note 3) | 7.35 |
| 6. Ceresin | 5 |
| 7. Isotridecyl isononanoate | 2 |
| 8. Titanium dioxide treated with silicone (Note 4) | 7 |
| 9. Black iron oxide treated with silicone (Note 4) | 0.1 |
| 10. Red ocher treated with silicone (Note 4) | 0.3 |
| 11. Yellow iron oxide treated with silicone (Note 4) | 0.6 |
| 12. KP-545 (Note 5) | 0.3 |
| 13. Butylene glycol | 7 |
| 14. Glycerin | 5 |
| 15. Sodium citrate | 0.2 |
| 16. Sodium chloride | 1 |
| 17. Water | Remaining amount |
| Total | 100 |

| | |
|---|---|
| (Note 1) Lauryl PEG-9 polydimethylsiloxyethyl dimethicone (labeling name), manufactured by Shin-Etsu Chemical Co., Ltd. (Note 2) 45% titanium dioxide dispersion, manufactured by Shin-Etsu Chemical Co., Ltd. (Note 3) Diphenylsiloxy phenyl trimethicone (labeling name), manufactured by Shin-Etsu Chemical Co., Ltd. (Note 4) Treated with KF-9909, manufactured by Shin-Etsu Chemical Co., Ltd. (Note 5) Acrylates/dimethicone copolymer (labeling name), manufactured by Shin-Etsu Chemical Co., Ltd. | |

The resulting W/O solid foundation had a good feeling when used, good applicability, and excellent storage stability.

### [Formulation Example 12]

### Stick concealer

### <Preparation of the cosmetic>

A: Components 1 to 7 were homogeneously mixed at 95°C and then filled into a stick container to obtain the stick concealer.

| Components | Mass (%) |
|---|---|
| 1. Organosiloxane Mixture 2 | 6 |
| 2. KSP-411 (Note 1) | 10 |
| 3. KMP-592 (Note 2) | 16 |
| 4. Dimethicone (6cs) | 22 |
| 5. Triethylhexanoin | 30 |
| 6. Microcrystalline wax | 6 |
| 7. Ceresin | 10 |
| Total | 100 |

| | |
|---|---|
| (Note 1) Polysilicone-1 crosspolymer (labeling name), manufactured by Shin-Etsu Chemical Co., Ltd. (Note 2) Methyl/phenyl polysilsesquioxane (labeling name), manufactured by Shin-Etsu Chemical Co., Ltd. | |

The resulting stick concealer had a good feeling when used, good applicability, and excellent storage stability.

### [Formulation Example 13]

### Lipstick

### <Preparation of the cosmetic>

A: Components 11 to 18 were dispersed using a roll.
B: Components 1 to 9 were homogeneously mixed at 95°C.
C: The mixture A and Component 10 were added to the mixture B, and the resultant was cooled to obtain the lipstick.

| Components | Mass (%) |
|---|---|
| 1. Crosslinked organosiloxane polymer of Example 1 | 0.5 |
| 2. Polyethylene | 7 |
| 3. Microcrystalline wax | 3 |
| 4. KP-561P (Note 1) | 10 |
| 5. Triethylhexanoin | 17.5 |
| 6. Neopentyl glycol diethylhexanoate | 15 |
| 7. Neopentyl glycol dicaprate | 7 |
| 8. Hydrogenated polyisobutene | Remaining amount |
| 9. KF-54HV (Note 2) | 8 |
| 10. Pearl pigment | 5 |
| 11. Mica | 0.3 |
| 12. CI 15850:1 | 0.1 |
| 13. CI 19140 | 0.3 |
| 14. Titanium dioxide treated with KP-574 (Note 3) | 0.8 |
| 15. Black iron oxide treated with KP-574 (Note 3) | 0.06 |
| 16. CI 15850 | 0.04 |
| 17. Red ocher treated with KP-574 (Note 3) | 0.2 |
| 18. Polyglyceryl-2 triisostearate | 1.2 |
| Total | 100 |

| | |
|---|---|
| (Note 1) Acrylates/stearyl acrylate/dimethicone methacrylate copolymer (labeling name), manufactured by Shin-Etsu Chemical Co., Ltd. (Note 2) Diphenyl dimethicone (labeling name), manufactured by Shin-Etsu Chemical Co., Ltd. (Note 3) Acrylates/tridecyl acrylate/triethoxysilylpropyl methacrylate/dimethicone methacrylate copolymer (labeling name), manufactured by Shin-Etsu Chemical Co., Ltd. | |

The resulting lipstick had a good feeling when used, good applicability, and excellent storage stability.

### [Formulation Example 14]

### Mascara

### <Preparation of the cosmetic>

A: Components 1 to 7 were homogeneously mixed at 90°C.
B: Components 8 to 13 were added to the mixture A, and the resultant was homogeneously mixed to obtain the mascara.

| Components | Mass (%) |
|---|---|
| 1. Organosiloxane Mixture 4 | 5 |
| 2. TSPL-30-ID (Note 1) | 10 |
| 3. Isododecane | Remaining amount |
| 4. Dextrin palmitate | 2 |
| 5. Ceresin | 7 |
| 6. Microcrystalline wax | 5 |
| 7. Disteardimonium hectorite | 5 |
| 8. Black iron oxide treated with alkylsilane (Note 2) | 5 |
| 9. Talc treated with alkylsilane (Note 2) | 5 |
| 10. KMP-590 (Note 3) | 5 |
| 11. Silicone-branched polyether-modified silicone (Note 4) | 1.2 |
| 12. Propylene carbonate | 1.6 |
| 13. Phenoxyethanol | 0.2 |
| Total | 100 |

| | |
|---|---|
| (Note 1) Trimethylsiloxysilylcarbamoyl pullulan (labeling name), manufactured by Shin-Etsu Chemical Co., Ltd. (Note 2) Treated with AES-3083, manufactured by Shin-Etsu Chemical Co., Ltd. (Note 3) Polymethylsilsesquioxane (labeling name), manufactured by Shin-Etsu Chemical Co., Ltd. (Note 4) PEG-9 polydimethylsiloxyethyl dimethicone (labeling name), manufactured by Shin-Etsu Chemical Co., Ltd. | |

The resulting mascara had a good feeling when used, good applicability, and excellent storage stability.

### [Formulation Example 15]

### Powder foundation

### <Preparation of the cosmetic>

A: Components 1 to 5 were heated to 50°C, homogeneously mixed, and then cooled to room temperature.
B: Components 6 to 14 were homogeneously mixed.
C: The mixture A was added to the mixture B, and the resultant was homogeneously mixed using a Henschel-Mixer. The resulting powder was passed through a mesh and then molded into a metal plate using a mold to obtain the powder foundation.

| Components | Mass (%) |
|---|---|
| 1. Ethylhexyl methoxycinnamate | 4 |
| 2. KF-56A (Note 1) | 4.5 |
| 3. Triethylhexanoin | 1.5 |
| 4. KP-561P (Note 2) | 1 |
| 5. Organosiloxane Mixture 2 | 1 |
| 6. Mica treated with silicone (Note 3) | 30 |
| 7. Barium sulfate | 10 |
| 8. KSP-300 (Note 4) | 5 |
| 9. KSP-100 (Note 5) | 4 |
| 10. Talc treated with silicone (Note 3) | Remaining amount |
| 11. Titanium dioxide treated with silicone (Note 3) | 6 |
| 12. Yellow iron oxide treated with silicone (Note 3) | 0.5 |
| 13. Red iron oxide treated with silicone (Note 3) | 0.2 |
| 14. Black iron oxide treated with silicone (Note 3) | 0.1 |
| Total | 100 |

| | |
|---|---|
| (Note 1) Diphenylsiloxy phenyl trimethicone (labeling name), manufactured by Shin-Etsu Chemical Co., Ltd. (Note 2) Acrylates/stearyl acrylate/dimethicone methacrylate copolymer (labeling name), manufactured by Shin-Etsu Chemical Co., Ltd. (Note 3) Treated with KF-9909, manufactured by Shin-Etsu Chemical Co., Ltd. (Note 4) Diphenyl dimethicone/vinyl diphenyl dimethicone/silsesquioxane crosspolymer (labeling name), manufactured by Shin-Etsu Chemical Co., Ltd. (Note 5) Vinyl dimethicone/methicone silsesquioxane crosspolymer (labeling name), manufactured by Shin-Etsu Chemical Co., Ltd. | |

The resulting powder foundation had a good feeling when used, good applicability, and excellent storage stability.

### [Formulation Example 16]

### Hair oil

### <Preparation of the cosmetic>

A: Components 1 to 7 were homogeneously mixed to obtain the hair oil.

| Components | Mass (%) |
|---|---|
| 1. Organosiloxane Mixture 3 | 3 |
| 2. KF-56A (Note 1) | 7 |
| 3. Diethylhexyl succinate | 10 |
| 4. X-21-5613 (Note 2) | 1.5 |
| 5. Tocopherol | 0.1 |
| 6. Fragrance | 0.1 |
| 7. Light liquid isoparaffin | Remaining amount |
| Total | 100 |

| | |
|---|---|
| (Note 1) Diphenylsiloxy phenyl dimethicone (labeling name), manufactured by Shin-Etsu Chemical Co., Ltd. (Note 2) Dimethiconol (labeling name), manufactured by Shin-Etsu Chemical Co., Ltd. | |

The resulting hair oil had a good feeling when used, good applicability, and excellent storage stability.

### [Formulation Example 17]

### Hair treatment product

### <Preparation of the cosmetic>

A: Components 6 to 9 were heated to 70°C and homogeneously mixed.
B: Components 1 to 5 were heated to 70°C and homogeneously mixed.
C: The mixture B was added to the mixture A, the resultant was emulsified and cooled, then Components 10 and 11 were added to obtain the hair treatment product.

| Components | Mass (%) |
|---|---|
| 1. Crosslinked organosiloxane polymer of Example 4 | 0.5 |
| 2. Cetyl alcohol | 2 |
| 3. Cetyl ethylhexanoate | 3 |
| 4. Butyl parahydroxybenzoate | 0.1 |
| 5. KF-56A (Note 1) | 1.5 |
| 6. Behentrimonium chloride | 1 |
| 7. Propylene glycol | 5 |
| 8. Hydroxyethylcellulose | 0.1 |
| 9. Water | Remaining amount |
| 10. X-52-2328 (Note 2) | 4 |
| 11. Fragrance | Appropriate amount |
| Total | 100 |

| | |
|---|---|
| (Note 1) Diphenylsiloxy phenyl dimethicone (labeling name), manufactured by Shin-Etsu Chemical Co., Ltd. (Note 2) Aminopropyl dimethicone (labeling name), manufactured by Shin-Etsu Chemical Co., Ltd. | |

The resulting hair treatment product had a good feeling when used, good applicability, and excellent storage stability.

### [Formulation Example 18]

### W/O sunscreen

### <Preparation of the cosmetic>

A: Components 1 to 9 were heated and homogeneously mixed.
B: Components 11 to 14 were homogeneously mixed.
C: The mixture B was added to the mixture A, the resultant was emulsified, then Component 10 was added, and the resultant was homogeneously mixed to obtain the W/O sunscreen.

| Components | Mass (%) |
|---|---|
| 1. Crosslinked organosiloxane polymer of Example 2 | 0.5 |
| 2. KSG-240 (Note 1) | 0.5 |
| 3. Organosiloxane Mixture 5 | 3 |
| 3. KF-6048 (Note 2) | 2 |
| 4. Ethylhexyl methoxycinnamate | 7.5 |
| 5. Octocrylene | 2.5 |
| 6. Diethylamino hydroxybenzoyl hexyl benzoate | 3 |
| 7. Ethylhexyl salicylate | 3 |
| 8. Homosalate | 4 |
| 9. KF-56A (Note 3) | 2.5 |
| 10. SPD-T5 (Note 4) | 12 |
| 11. Ethanol | 6 |
| 12. Sodium citrate | 0.5 |
| 13. Sodium chloride | 0.5 |
| 14. Water | Remaining amount |
| Total | 100 |

| | |
|---|---|
| (Note 1) Mixture of 20% dimethicone/PEG-10/15 crosspolymer (labeling name) and 80% cyclopentasiloxane (labeling name), manufactured by Shin-Etsu Chemical Co., Ltd. (Note 2) Cetyl PEG/PPG-10/1 dimethicone (labeling name), manufactured by Shin-Etsu Chemical Co., Ltd. (Note 3) Diphenylsiloxy phenyl dimethicone (labeling name), manufactured by Shin-Etsu Chemical Co., Ltd. (Note 4) 40% titanium dioxide dispersion, manufactured by Shin-Etsu Chemical Co., Ltd. | |

The resulting W/O sunscreen had a good feeling when used, good applicability, good transparency, and excellent storage stability.

### [Formulation Example 19]

### W/O liquid foundation

### <Preparation of the cosmetic>

A: Components 9 to 15 were dispersed using a three-roll mill.
B: Components 1 to 8 were homogeneously mixed.
C: Components 16 to 19 were homogeneously mixed.
D: The mixture C was added to the mixture B, the resultant was emulsified, and then the mixture A was added to obtain the W/O liquid foundation.

| Components | Mass (%) |
|---|---|
| 1. Organosiloxane Mixture 5 | 3.5 |
| 2. Crosslinked organosiloxane polymer of Example 1 | 5 |
| 3. KF-6038 (Note 2) | 2.5 |
| 4. Organically modified clay mineral | 0.8 |
| 5. Dimethicone (2cs) | 21 |
| 6. Ethylhexyl methoxycinnamate | 7.5 |
| 7. Diethylamino hydroxybenzoyl hexyl benzoate | 2.5 |
| 8. KSP-101 (Note 3) | 2 |
| 9. TMF-1.5 (Note 4) | 6 |
| 10. KF-6105 (Note 5) | 0.5 |
| 11. KTP-09W (Note 6) | 10 |
| 12. KTP-09R (Note 6) | 0.2 |
| 13. KTP-09Y (Note 6) | 0.5 |
| 14. KTP-09B (Note 6) | 0.1 |
| 15. Zinc oxide fine particles treated with alkylsilane | 10 |
| 16. Butylene glycol | 5 |
| 17. Sodium citrate | 0.2 |
| 18. Sodium chloride | 1 |
| 19. Water | Remaining amount |
| Total | 100 |

| | |
|---|---|
| (Note 1) Mixture of 25% dimethicone/PEG-10/15 crosspolymer (labeling name) and 75% dimethicone, manufactured by Shin-Etsu Chemical Co., Ltd. (Note 2) Cetyl PEG/PPG-10/1 dimethicone (labeling name), manufactured by Shin-Etsu Chemical Co., Ltd. (Note 3) Vinyl dimethicone/methicone silsesquioxane crosspolymer (labeling name), manufactured by Shin-Etsu Chemical Co., Ltd. (Note 4) Methyl trimethicone (labeling name), manufactured by Shin-Etsu Chemical Co., Ltd. (Note 5) Lauryl polyglyceryl-3 polydimethylsiloxyethyl dimethicone (labeling name), manufactured by Shin-Etsu Chemical Co., Ltd. (Note 6) Inorganic color pigments treated with KF-9909; W (white), R (red), Y (yellow), and B (black), manufactured by Shin-Etsu Chemical Co., Ltd. | |

The resulting W/O liquid foundation had a good feeling when used, good applicability, and excellent storage stability.

### [Formulation Example 20]

### W/O liquid foundation

### <Preparation of the cosmetic>

A: Components 9 to 15 were dispersed using a three-roll mill.
B: Components 1 to 8 were homogeneously mixed.
C: Components 16 to 19 were homogeneously mixed.
D: The mixture C was added to the mixture B, the resultant was emulsified, and then the mixture A was added to obtain the W/O liquid foundation.

| Components | Mass (%) |
|---|---|
| 1. Organosiloxane Mixture 5 | 3.5 |
| 2. Crosslinked organosiloxane polymer of Example 4 | 1 |
| 3. KF-6028 (Note 1) | 2.5 |
| 4. Organically modified clay mineral | 0.8 |
| 5. Dimethicone (2cs) | 25 |
| 6. Ethylhexyl palmitate | 7 |
| 7. Caprylic/capric triglyceride | 5 |
| 8. KSP-101 (Note 2) | 2 |
| 9. KF-56A (Note 3) | 7 |
| 10. KF-6106 (Note 4) | 1 |
| 11. KTP-09W (Note 5) | 10 |
| 12. KTP-09R (Note 5) | 0.2 |
| 13. KTP-09Y (Note 5) | 0.5 |
| 14. KTP-09B (Note 5) | 0.1 |
| 15. Titanium dioxide fine particles treated with metallic soap | 10 |
| 16. Butylene glycol | 5 |
| 17. Sodium citrate | 0.2 |
| 18. Sodium chloride | 1 |
| 19. Water | Remaining amount |
| Total | 100 |

| | |
|---|---|
| (Note 1) PEG-9 polydimethylsiloxyethyl dimethicone (labeling name), manufactured by Shin-Etsu Chemical Co., Ltd. (Note 2) Vinyl dimethicone/methicone silsesquioxane crosspolymer (labeling name), manufactured by Shin-Etsu Chemical Co., Ltd. (Note 3) Diphenylsiloxy phenyl dimethicone (labeling name), manufactured by Shin-Etsu Chemical Co., Ltd. (Note 4) Lauryl polyglyceryl-3 polydimethylsiloxyethyl dimethicone (labeling name), manufactured by Shin-Etsu Chemical Co., Ltd. (Note 5) Inorganic color pigments treated with KF-9909; W (white), R (red), Y (yellow), and B (black), manufactured by Shin-Etsu Chemical Co., Ltd. | |

The resulting W/O liquid foundation had a good feeling when used, good applicability, and excellent storage stability.

### [Formulation Example 21]

### Suncare stick

### <Preparation of the cosmetic>

A: Components 1 to 14 were homogeneously mixed at 110°C and then filled into a stick container to obtain the suncare stick.

| Components | Mass (%) |
|---|---|
| 1. Crosslinked organosiloxane polymer of Example 3 | 2 |
| 2. KF-56A (Note 1) | Remaining amount |
| 3. KMP-592 (Note 2) | 0.5 |
| 4. Diisopropyl sebacate | 10 |
| 5. Ethylhexyl methoxycinnamate | 7.5 |
| 6. Octocrylene | 2.5 |
| 7. Diethylamino hydroxybenzoyl hexyl benzoate | 5 |
| 8. Ethylhexyl salicylate | 5 |
| 9. Bis-ethylhexyloxyphenol methoxyphenyl triazine | 3 |
| 10. Polysilicone-15 | 2 |
| 11. Homosalate | 10 |
| 12. Dibutyl lauroyl glutamide | 3.5 |
| 13. Octyldodecanol | 10 |
| 14. Mineral oil | 2 |
| Total | 100 |

| | |
|---|---|
| (Note 1) Diphenylsiloxy phenyl dimethicone (labeling name), manufactured by Shin-Etsu Chemical Co., Ltd. (Note 2) Methyl/phenyl polysilsesquioxane (labeling name), manufactured by Shin-Etsu Chemical Co., Ltd. | |

The resulting suncare stick had a good feeling when used, good applicability, good transparency, and excellent storage stability.

### [Formulation Example 22]

### W/O beauty stick

### <Preparation of the cosmetic>

A: Components 1 to 6 were homogeneously mixed at 90°C.
B: Components 7 to 12 were homogeneously mixed at 85°C.
C: The mixture B was added to the mixture A, and the resultant was emulsified and filled into a stick container to obtain the W/O beauty stick.

| Components | Mass (%) |
|---|---|
| 1. Crosslinked organosiloxane polymer of Example 6 | 1 |
| 2. Organosiloxane Mixture 4 | 5 |
| 3. KF-6105 (Note 1) | 0.5 |
| 4. Limnanthes alba (meadowfoam) seed oil | 2 |
| 5. KF-56A (Note 2) | 12 |
| 6. Ceresin | 10 |
| 7. Butylene glycol | 7 |
| 8. Sodium PCA | 2 |
| 9. Diglycerin | 3 |
| 10. Sodium citrate | 0.2 |
| 11. Magnesium sulfate | 1 |
| 12. Water | Remaining amount |
| Total | 100 |

| | |
|---|---|
| (Note 1) Lauryl polyglyceryl-3 polydimethylsiloxyethyl dimethicone (labeling name), manufactured by Shin-Etsu Chemical Co., Ltd. (Note 2) Diphenylsiloxy phenyl trimethicone (labeling name), manufactured by Shin-Etsu Chemical Co., Ltd. | |

The resulting W/O beauty stick had a good feeling when used, good applicability, and excellent storage stability.

### [Formulation Example 23]

### W/O stick foundation

### <Preparation of the cosmetic>

A: Components 12 to 17 were dispersed using a three-roll mill.
B: Components 1 to 11 were dissolved at 90°C, then the mixture A was added, and the resultant was homogeneously mixed.
C: Components 18 to 23 were homogeneously mixed.
D: The mixture C was added to the mixture B, and the resultant was emulsified and filled into a stick container to obtain the W/O stick foundation.

| Components | Mass (%) |
|---|---|
| 1. Organosiloxane Mixture 5 | 5 |
| 2. Crosslinked organosiloxane polymer of Example 1 | 1 |
| 3. KF-6038 (Note 2) | 2 |
| 4. KF-995 (Note 3) | 12 |
| 5. Stearoyl inulin | 3 |
| 6. Synthetic wax | 7 |
| 7. Butyl methoxydibenzoylmethane | 2 |
| 8. Ethylhexyl salicylate | 4 |
| 9. Bis-ethylhexyloxyphenol methoxyphenyl triazine | 2 |
| 10. Polysilicone-15 | 2 |
| 11. Homosalate | 5 |
| 12. Isotridecyl isononanoate | 2 |
| 13. KP-578 (Note 4) | 0.3 |
| 14. Titanium dioxide treated with silicone (Note 5) | 7 |
| 15. Black iron oxide treated with silicone (Note 5) | 0.1 |
| 16. Red ocher treated with silicone (Note 5) | 0.3 |
| 17. Yellow iron oxide treated with silicone (Note 5) | 0.6 |
| 18. Butylene glycol | 7 |
| 19. Glycerin | 5 |
| 20. Sodium citrate | 0.2 |
| 21. Sodium chloride | 1 |
| 22. Disodium edetate | 0.1 |
| 23. Water | Remaining amount |
| Total | 100 |

| | |
|---|---|
| (Note 1) Mixture of 25% dimethicone/polyglycerin-3 crosspolymer (labeling name) and 75% dimethicone, manufactured by Shin-Etsu Chemical Co., Ltd. (Note 2) Lauryl PEG-9 polydimethylsiloxyethyl dimethicone (labeling name), manufactured by Shin-Etsu Chemical Co., Ltd. (Note 3) Cyclopentasiloxane (labeling name), manufactured by Shin-Etsu Chemical Co., Ltd. (Note 4) Acrylates/ethylhexyl acrylate/dimethicone methacrylate copolymer (labeling name), manufactured by Shin-Etsu Chemical Co., Ltd. (Note 5) Treated with KF-9901, manufactured by Shin-Etsu Chemical Co., Ltd. | |

The resulting W/O stick foundation had a good feeling when used, good applicability, and excellent storage stability.

### [Formulation Example 24]

### Mousse foundation

### <Preparation of the cosmetic>

A: Components 9 to 16 were dispersed using a three-roll mill.
B: Components 1 to 8 were homogeneously mixed.
C: The mixture A was added to the mixture B, and the resultant was homogeneously mixed to obtain the mousse foundation.

| Components | Mass (%) |
|---|---|
| 1. KSG-16 (Note 1) | 20 |
| 2. Crosslinked organosiloxane polymer of Example 2 | 2 |
| 3. KF-7312T (Note 2) | 8 |
| 4. Neopentyl glycol diethylhexanoate | 4 |
| 5. Dimethicone (2cs) | Remaining amount |
| 6. Simmondsia chinensis (jojoba) seed oil | 0.5 |
| 7. KSP-105 (Note 3) | 2 |
| 8. Polymethyl methacrylate | 6 |
| 9. Dimethicone (6cs) | 10 |
| 10. Titanium dioxide treated with silicone (Note 4) | 5 |
| 11. Titanium dioxide fine particles treated with metallic soap | 10 |
| 12. Red iron oxide treated with silicone (Note 4) | 0.2 |
| 13. Yellow iron oxide treated with silicone (Note 4) | 0.5 |
| 14. Black iron oxide treated with silicone (Note 4) | 0.1 |
| 15. Talc treated with silicone (Note 4) | 3 |
| 16. Black mica treated with silicone (Note 4) | 5 |
| Total | 100 |

| | |
|---|---|
| (Note 1) Mixture of 25% dimethicone/vinyl dimethicone crosspolymer (labeling name) and 75% dimethicone (labeling name), manufactured by Shin-Etsu Chemical Co., Ltd. (Note 2) Trimethylsiloxysilicate (labeling name), manufactured by Shin-Etsu Chemical Co., Ltd. (Note 3) Vinyl dimethicone/methicone silsesquioxane crosspolymer (labeling name), manufactured by Shin-Etsu Chemical Co., Ltd. (Note 4) Treated with KF-9908, manufactured by Shin-Etsu Chemical Co., Ltd. | |

The resulting mousse foundation had a good feeling when used, good applicability, and excellent storage stability.

### [Formulation Example 25]

### Mousse foundation

### <Preparation of the cosmetic>

A: Components 12 to 19 were dispersed using a three-roll mill.
B: Components 1 to 11 were homogeneously mixed.
C: The mixture A was added to the mixture B, and the resultant was homogeneously mixed to obtain the mousse foundation.

| Components | Mass (%) |
|---|---|
| 1. KSG-18A (Note 1) | 8 |
| 2. Crosslinked organosiloxane polymer of Example 2 | 3 |
| 3. KF-7312J (Note 2) | 8 |
| 4. Cyclopentasiloxane | Remaining amount |
| 5. Silica dimethyl silylate | 0.5 |
| 6. Ethylhexyl methoxycinnamate | 7 |
| 7. Diethylamino hydroxybenzoyl hexyl benzoate | 2 |
| 8. Bis-ethylhexyloxyphenol methoxyphenyl triazine | 1 |
| 9. Homosalate | 5 |
| 10. KSP-105 (Note 3) | 2 |
| 11. Polymethyl methacrylate | 6 |
| 12. KF-56A (Note 4) | 10 |
| 13. Titanium dioxide treated with alkylsilane (Note 5) | 5 |
| 14. Titanium dioxide fine particles treated with metallic soap | 8 |
| 15. Red iron oxide treated with alkylsilane (Note 5) | 0.2 |
| 16. Yellow iron oxide treated with alkylsilane (Note 5) | 0.5 |
| 17. Black iron oxide treated with alkylsilane (Note 5) | 0.1 |
| 18. Talc treated with alkylsilane (Note 5) | 3 |
| 19. Black mica treated with alkylsilane (Note 5) | 4 |
| Total | 100 |

| | |
|---|---|
| (Note 1) Mixture of 15% dimethicone/phenyl vinyl dimethicone crosspolymer (labeling name) and 85% dimethicone (labeling name), manufactured by Shin-Etsu Chemical Co., Ltd. (Note 2) Trimethylsiloxysilicate (labeling name), manufactured by Shin-Etsu Chemical Co., Ltd. (Note 3) Vinyl dimethicone/methicone silsesquioxane crosspolymer (labeling name), manufactured by Shin-Etsu Chemical Co., Ltd. (Note 4) Diphenylsiloxy phenyl trimethicone (labeling name), manufactured by Shin-Etsu Chemical Co., Ltd. (Note 5) Treated with AES-3083, manufactured by Shin-Etsu Chemical Co., Ltd. | |

The resulting mousse foundation had a good feeling when used, good applicability, and excellent storage stability.

### [Formulation Example 26]

### Gel colored eyeshadow

### <Preparation of the cosmetic>

A: Components 1 to 3 were homogeneously mixed at 80°C.
B: The mixture A and Components 4 to 5 were homogeneously mixed at 60°C.
C: The mixture B and Components 6 to 11 were homogeneously mixed to obtain the gel colored eyeshadow.

| Components | Mass (%) |
|---|---|
| 1. Isotridecyl isononanoate | 30 |
| 2. Squalane | Remaining amount |
| 3. Dextrin palmitate | 10 |
| 4. Crosslinked organosiloxane polymer of Example 2 | 3 |
| 5. Silica dimethyl silylate | 0.1 |
| 6. KSP-100 (Note 1) | 8 |
| 7. KMP-590 (Note 2) | 2 |
| 8. Barium sulfate | 5 |
| 9. Silicone-treated synthetic mica (Note 3) | 13 |
| 10. Glass powder | 7 |
| 11. Polyethylene terephthalate (and) aluminum | 4.5 |
| Total | 100 |

| | |
|---|---|
| (Note 1) Vinyl dimethicone/methicone silsesquioxane crosspolymer (labeling name), manufactured by Shin-Etsu Chemical Co., Ltd. (Note 2) Polymethylsilsesquioxane (labeling name), manufactured by Shin-Etsu Chemical Co., Ltd. (Note 3) Treated with KP-574, manufactured by Shin-Etsu Chemical Co., Ltd. | |

The resulting gel colored eyeshadow had a good feeling when used, good applicability, and excellent storage stability.

### [Formulation Example 27]

### Colored eyeshadow

### <Preparation of the cosmetic>

A: Components 1 to 5 were homogeneously mixed.
B: Components 6 to 15 were homogeneously mixed.
C: The mixture A was added to the mixture B, and the resultant was homogeneously mixed using a Henschel-Mixer. The resulting powder was passed through a mesh and then molded into a metal plate using a mold to obtain the colored eyeshadow.

| Components | Mass (%) |
|---|---|
| 1. Diisostearyl malate | 3 |
| 2. KF-56A (Note 1) | 3 |
| 3. Triethylhexanoin | 2.5 |
| 4. Crosslinked organosiloxane polymer of Example 4 | 1 |
| 5. Petrolatum | 2 |
| 6. KMP-590 (Note 2) | 2 |
| 7. Mica treated with silicone (Note 3) | Remaining amount |
| 8. Talc treated with silicone (Note 3) | 25 |
| 9. Barium sulfate | 3 |
| 10. Synthetic fluorphlogopite | 10 |
| 11. Boron nitride | 2 |
| 12. KSP-300 (Note 4) | 4 |
| 13. Zinc myristate | 0.5 |
| 14. Sorbitan sesquiisostearate | 0.2 |
| 15. Glass-based pearl pigment | 12 |
| Total | 100 |

| | |
|---|---|
| (Note 1) Diphenylsiloxy phenyl trimethicone (labeling name), manufactured by Shin-Etsu Chemical Co., Ltd. (Note 2) Polymethylsilsesquioxane (labeling name), manufactured by Shin-Etsu Chemical Co., Ltd. (Note 3) Treated with KF-9909, manufactured by Shin-Etsu Chemical Co., Ltd. (Note 4) Diphenyl dimethicone/vinyl diphenyl dimethicone/silsesquioxane crosspolymer (labeling name), manufactured by Shin-Etsu Chemical Co., Ltd. | |

The resulting colored eyeshadow had a good feeling when used, good applicability, and excellent storage stability.

Note that the present invention is not limited to the embodiments above. The embodiments above are examples, and anything that has substantially the same configuration as the technical idea described in the claims of the present invention and achieves similar effects shall be included in the technical scope of the present invention.

## Claims

1. A cosmetic comprising a crosslinked organosiloxane polymer having a crosslinked structure including a carbon-silicon bond, which is non-fluid at 25 °C and has
a loss coefficient of 1.1 or less at a shear frequency of 1 Hz, a measurement temperature of 25 °C and a shear strain of 10 %, and
a loss coefficient of 0.9 to 1.4 at a shear frequency of 10 Hz, a measurement temperature of 25 °C and a shear strain of 10 %.

2. The cosmetic according to claim 1, wherein the crosslinked organosiloxane polymer is a product of a hydrosilylated reaction of one or more kinds of organohydrogen polysiloxanes with one or more kinds of alkenyl-group-containing organic compounds, wherein the organohydrogen polysiloxanes have 2 to 10,000 silicon atoms and 1.01 to 10 hydroxy groups per molecule of the organohydrogen polysiloxane, and the alkenyl-group-containing organic compounds preferably have a weight average molecular weight of 50 to 9,000,000 and 1.01 to 10 alkenyl groups per molecule of the alkenyl-group-containing compound.

3. The cosmetics according to claim 2, wherein the organohydrogen polysiloxane comprises (A1) an organohydrogen polysiloxane represented by the following general formula (1):
Mₐ₁M'ₐ₂D_{b1}D'_{b2}T_{c1}T'_{c2}Q_{d} (1)
wherein M is a siloxane unit represented by R¹₃SiO_{1/2};
M' is a siloxane unit represented by R¹₂HSiO_{1/2};
D is a siloxane unit represented by R¹₂SiO_{2/2};
D' is a siloxane unit represented by R¹HSiO_{2/2};
T is a siloxane unit represented by R¹SiO_{3/2};
T' is a siloxane unit represented by HSiO_{3/2}; and
Q is a siloxane unit represented by SiO_{4/2};
wherein R¹ is, independently of each other, a group selected from an alkyl group having 1 to 30 carbon atoms, an aryl group having 6 to 30 carbon atoms, and an aralkyl group having 7 to 30 carbon atoms; and a1, a2, b1, b2, c1, c2, and d satisfy 0 ≤ a1 ≤ 100, 0 ≤ a2 ≤ 10, 0 ≤ b1 ≤ 10,000, 0 ≤ b2 ≤ 10, 0 ≤ c1 ≤ 100, 0 ≤ c2 ≤ 10, and 0 ≤ d ≤ 50, respectively, as well as 2 ≤ a1 + a2 + b1 + b2 + c1 + c2 + d ≤ 10,000 and 1.01 ≤ a2 + b2 + c2 ≤ 10,
provided that the organohydrogen polysiloxane may optionally comprise an organohydrogen polysiloxane represented by the general formula (1) of which a2 + b2 + c2 is 1, in addition to the said organohydrogen polysiloxane represented by the general formula (1).

4. The cosmetic according to claim 2, wherein the alkenyl-group-containing organic compound is selected from the following components (A2), (A3), (A4), and (A5):
(A2) an alkenyl-group-having organopolysiloxane, represented by the following general formula (2):
Mₑ₁M"ₑ₂D_{f1}D"_{f2}T_{g1}T"_{g2}Qₕ (2)
wherein M, D, T, and Q are as described above;
M" is a siloxane unit represented by R¹₂R²SiO_{1/2};
D" is a siloxane unit represented by R¹R²SiO_{2/2}; and
T" is a siloxane unit represented by R²SiO_{3/2},
wherein R¹ is, independently of each other, a group selected from an alkyl group having 1 to 30 carbon atoms, an aryl group having 6 to 30 carbon atoms, and an aralkyl group having 7 to 30 carbon atoms; R² is an alkenyl group having 2 to 10 carbon atoms; and e1, e2, f1, f2, g1, g2, and h satisfy 0 ≤ e1 ≤ 100, 0 ≤ e2 ≤ 10, 0 ≤ f1 ≤ 100,000, 0 ≤ f2 ≤ 10, 0 ≤ g1 ≤ 100, 0 ≤ g2 ≤ 10, and 0 ≤ h ≤ 50, respectively, as well as 2 ≤ e1 + e2 + f1 + f2 + g1 + g2 + h ≤ 100,000 and 1.01 ≤ e2 + f2 + g2 ≤ 10,
provided that the alkenyl-group-having organopolysiloxane (A2) may optionally comprise an alkenyl-group-containing organopolysiloxane represented by the general formula (2) of which e2 + f2 + g2 is 1, in addition to the said alkenyl-group-having organopolysiloxane represented by the general formula (2);
(A3) an alkenyl-group-having oxyalkylene with alkenyl groups at both terminals, represented by the following general formula (3):
CH₂=CH-(CH₂)ᵢ-(OC₂H₄)ⱼ-(OCH(CH₃)CH₂)ₖ-O-(CH₂)ᵢ -CH=CH₂ (3)
wherein i is, independently of each other, an integer of 1 or more, 1 ≤ j ≤ 100 and 0 ≤ k ≤ 100; (A4) an alkene represented by the following general formula (4):
CH₂=CH-(CH₂)ᵣ-CH=CH₂ (4)
wherein 0 ≤ r ≤ 50; and
(A5) an allyl-group-containing polyglycerin derivative represented by the following general formula (5): 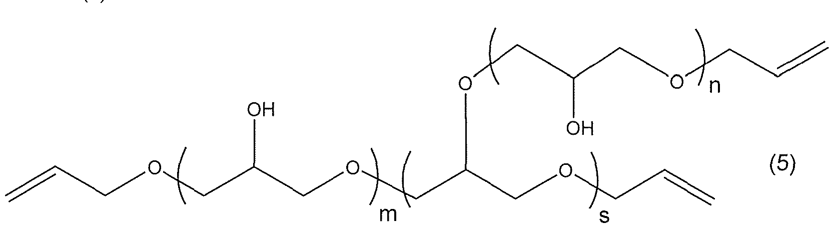 wherein 1 ≤ m ≤ 20, 1 ≤ n ≤ 20, and 0 ≤ s ≤ 10.

5. Cosmetic comprising a mixture of the cross-linked organosiloxane polymer according any one of claims 1 to 4 and an oil that is liquid at 25 °C, wherein the mixture has the form of liquid, paste or gel.
